(19) Europäisches Patentamt
European Patent Office
Office européen des brevets

(11) Publication number: **0 106 275 B1**

# (12) EUROPEAN PATENT SPECIFICATION

(45) Date of publication of patent specification: **13.03.91**

(51) Int. Cl.[5]: **C07D 211/90**, A61K 31/455, //C07D211/42

(21) Application number: **83109968.4**

(22) Date of filing: **05.10.83**

The file contains technical information submitted after the application was filed and not included in this specification

## (54) 1,4-Dihydropyridine derivatives.

(30) Priority: **15.10.82 JP 180616/82**
**27.01.83 JP 11719/83**
**03.06.83 JP 98899/83**

(43) Date of publication of application:
**25.04.84 Bulletin 84/17**

(45) Publication of the grant of the patent:
**13.03.91 Bulletin 91/11**

(84) Designated Contracting States:
**AT BE CH DE FR GB IT LI NL SE**

(56) References cited:
**EP-A- 0 063 365**
**EP-A- 0 071 819**
**FR-A- 2 376 137**
**GB-A- 1 383 625**
**GB-A- 2 014 134**

**PHARMAZIE IN UNSERER ZEIT, 2nd ed. (1973), page 73-77**

**D A Z, 116. Jahrg., Nr. 24, pages 849-51**

**D A Z, 124. Jahrg., Nr. 14, pages 685-91**

(73) Proprietor: **KYOWA HAKKO KOGYO CO., LTD.**
**Ohtemachi Bldg., 6-1 Ohtemachi I-chome**
**Chiyoda-ku Tokyo 100(JP)**

(72) Inventor: **Muto, Kenji**
**1188, Shimotogari Nagaizumi-cho**
**Sunto-gun Shizuoka-ken(JP)**
Inventor: **Kuroda, Tokuyuki**
**108-11, Futatsuya**
**Susono-shi Shizuoka-ken(JP)**
Inventor: **Karasawa, Akira**
**411-3, Shimonagakubo Nagaizumi-cho**
**Sunto-gun Shizuoka-ken(JP)**
Inventor: **Yamada, Koji**
**1309-3, Sano**
**Sussono-shi Shizooka-ken(JP)**
Inventor: **Nakamizo, Nobuhiro**
**1704-22, Honmachida**
**Machida-shi Tokyo(JP)**

(74) Representative: **Casalonga, Axel et al**
**BUREAU D.A. CASALONGA - JOSSE Morassistrasse 8**
**W-8000 München 5(DE)**

**EP 0 106 275 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

## Description

The present invention relates to 1,4-dihydropyridine derivatives. More particularly, the present invention relates to (±)-2,6-dimethyl-4-(3-nitrophenyl)-1,4-dihydropyridine-3,5-dicarboxylic acid-3-(1-benzyl-3-piperidyl) ester-5-methyl ester, the melting point of the hydrochloride of which is 196° C to 202° C [referred to as (±)-α-form], from among the diastereomers of 2,6-dimethyl-4-(3-nitrophenyl)-1,4-dihydropyridine-3,5-dicarboxylic acid-3-(1-benzyl-3-piperidyl) ester-5-methyl ester represented by the formula I:

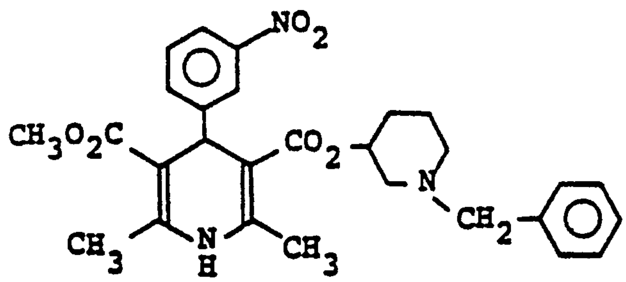

(I)

and an optical isomer having an optical rotation degree of (+) [referred to as (+)-α-form] from among (±)-α-form or a salt thereof.

The present compounds have effects to depress the blood pressure and to dilate coronary artery and peripheral blood vessels and are compounds useful as drugs for cardiovascular disease, such as hypotensives and vasodilators.

Also, (±)-2,6-dimethyl-4-(3-nitrophenyl)-1,4-dihydropyridine-3,5-dicarboxylic acid-3-(1-benzyl-3-piperidyl) ester-5-methyl ester, the hydrochloride of which has melting points of 236° C to 242° C is referred to as (±)-β-form.

Next, the processes for producing (±)-α-form are shown below.

Process 1:

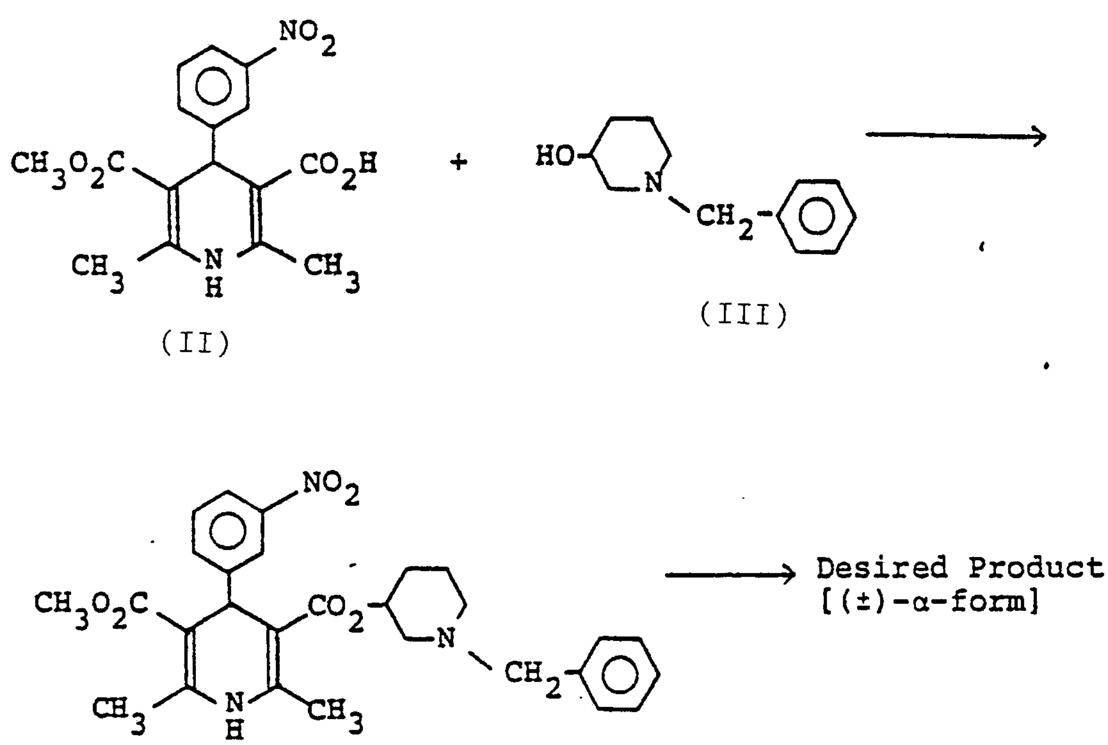

(I){Diastereomer [(±)-α-form. (±)-β-form]]

Esterification of the compound represented by the formula II (hereinafter referred to as Compound II; further compounds represented by formulae III to XI are similarly referred to as Compounds III to XI) and Compound III is carried out in a conventional manner, e.g.

a) a process which comprises converting Compound II into the acid halide using a halogenating reagent and then reacting the acid halide with Compound III;

b) a process which comprises reacting Compound II with Compound III in the presence of the condensing agent such as N,N'-dicyclohexylcarbodiimide;

and the process a) using an inexpensive halogenating reagent (for example, thionyl chloride, phosphorus trichloride, phosphorus pentachloride, phosphorus oxychloride and phosphorus tribromide) is particularly advantageous.

The process a) using halogenating reagents is explained in further detail.

The process in which the halogenating reagent is thionyl chloride, is illustrated by the following equations:

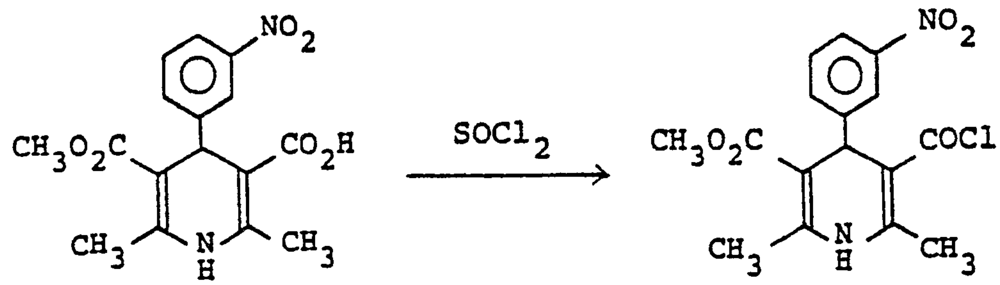


(II) (IV)

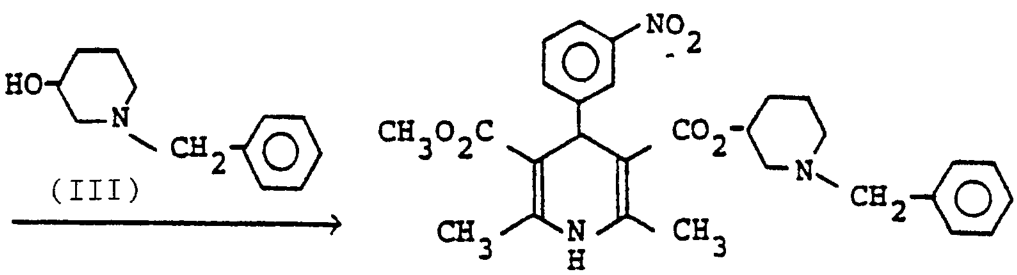

(III)

(I)( Diastereomer [(±)-α-form, (±)-β-form]}

→ Desired Product [(±)-α-form]

The reaction is carried out in the presence or absence of halogenated hydrocarbons such as dichloromethane, chloroform, carbon tetrachloride and chlorobenzene; aromatic hydrocarbons such as benzene and toluene; ethers such as tetrahydrofuran and dioxan; aprotic polar solvents such as acetonitrile, N,N-dimethylformamide and hexamethylphosphoric triamide; amines such as pyridine and triethyl amine. The reaction is preferably carried out using thionyl chloride as a halogenating reagent in the presence of N,N-dimethylformamide or hexamethylphosphoric triamide, with or without the aforesaid solvents alone or in combination.

The molar ratio of the carboxylic acid to thionyl chloride is in the range of 1.0 : 0.8 - 1.0 : 2.0, preferably 1.0 : 0.9 - 1.0 : 1.2.

The molar ratio of thionyl chloride to N,N-dimethylformamide or hexamethylphosphoric triamide is 1 : 1 - 1: 100, preferably 1 : 5 - 1 : 50.

The reaction is carried out at temperatures of -70° C to 100° C, preferably -20° C to 50° C.

Then, the resulting Compound IV (which may not be isolated) is reacted with Compound III to obtain diastereomers {(±)-α-form and (±)-β-form} represented by the formula I.

As the solvent, the same solvent as employed for preparation of Compound IV from Compound II is employed.

The molar ratio of Compound II to Compound III is in the range of 1.0 : 0.8 - 1.0 : 2.0, preferably 1.0 : 0.9 - 1.0 : 1.2 and the reaction temperature is -70° C to 100° C, preferably -20° C to 50° C.

Process 2:

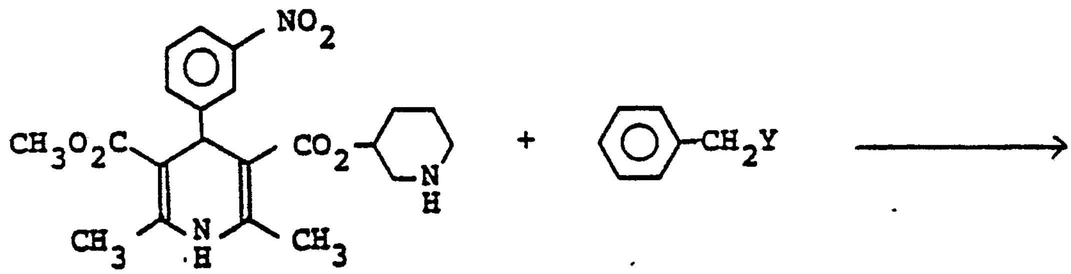

(V) (VI)

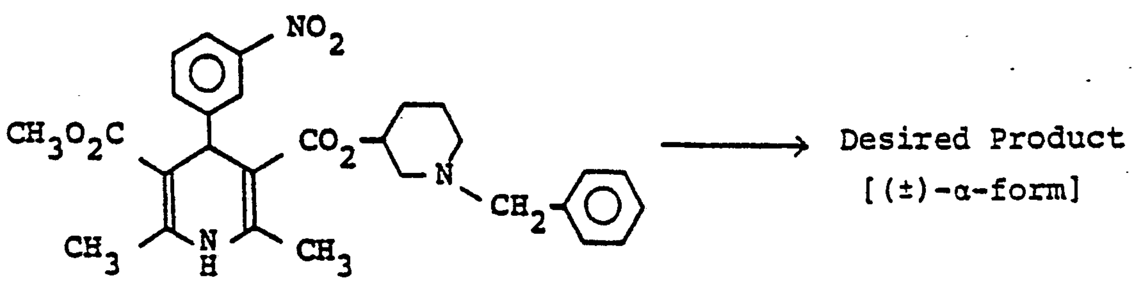


(I){ Diastereomer [(±)-α-form, (±)-β-form]} wherein Y represents a halogen atom.

Compound V which is employed as a starting material in this process is obtained by the process illustrated by the following reaction equations, in case that a halogenating reagent is thionyl chloride.

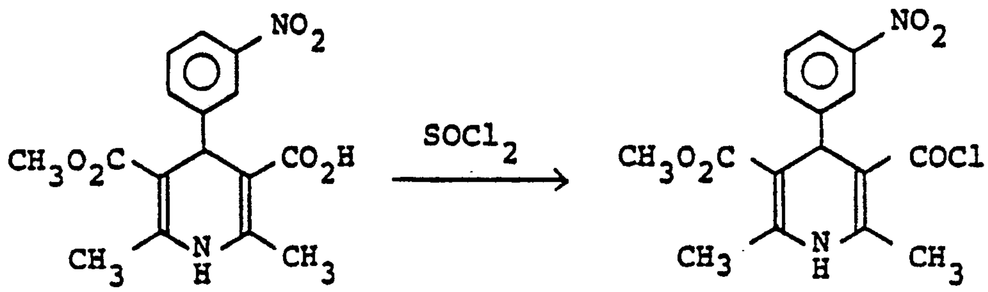


(II) (IV)

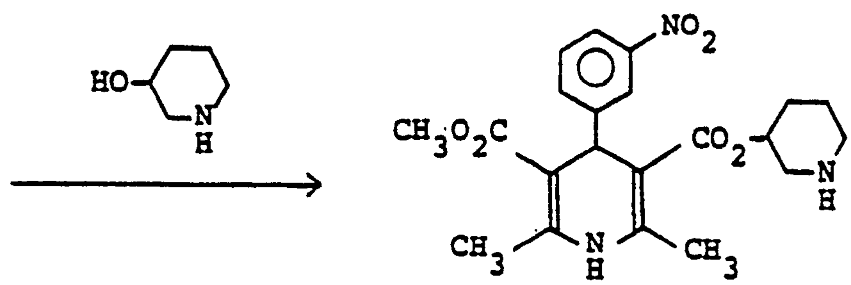

(V)

The molar ratio of Compound V to Compound VI is in the range of 1.0 : 0.5 - 1.0 : 4.0, preferably 1.0 : 0.9 - 1.0 : 2.0.

The reaction is carried out in the presence or absence of alcohols such as isopropanol and isobutanol; aromatic hydrocarbons such as benzene and toluene; halogenated hydrocarbons such as chloroform, carbon tetrachloride and chlorobenzene; ethers such as tetrahydrofuran, dioxan and dimethoxyethane; aprotic polar solvents such as acetonitrile and N,N-dimethylformamide; or water with or without adding organic bases such as triethyl amine, n-butyl amine and pyridine, or inorganic bases such as sodium hydroxide, potassium hydroxide and potassium carbonate to the reaction mixture at room temperature to 200° C, preferably 30° C to 150° C.

Process 3:

[a process in accordance with the process described in M. Iwanami et al., Chem. Pharm. Bull, 27, 1426 (1979)]

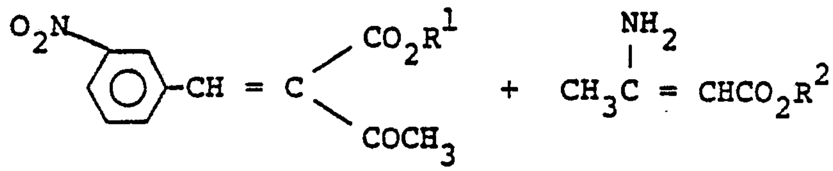

(VII) (VIII)

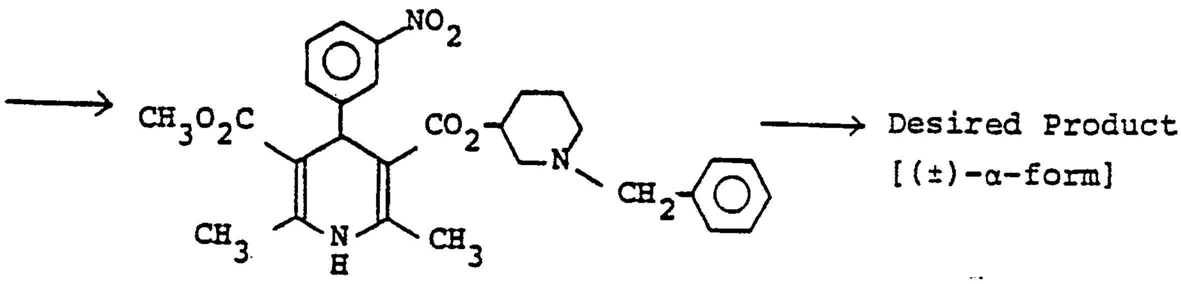


(I){Diastereomer [(±)-α-form, (±)-β-form]}
wherein one of $R_1$ and $R_2$ represents a methyl group, and the other represents a benzylpiperidyl group.

The molar ratio of Compound VII to Compound VIII is in the range of 1.0 : 0.8 - 1.0 : 4.0, preferably, 1.0 : 0.9 - 1.0 : 1.5.

The reaction is carried out in the presence or absence of alcohols such as methanol, ethanol and isopropanol; aromatic hydrocarbons such as benzene and toluene; halogenated hydrocarbons such as chloroform and carbon tetrachloride; ethers such as tetrahydrofuran, dioxan and dimethoxyethane; aprotic polar solvents such as acetonitrile and N,N-dimethylformamide; or water at room temperature to 150 °C, preferably 30 °C to 100 °C.

Process 4:

[a process in accordance with the process described in M. Iwanami et al., Chem. Pharm. Bull., 27, 1426 (1979)]

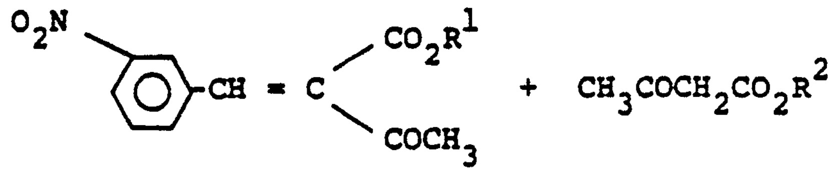

(VII) (IX)

+ $NH_3$ (or ammonium salt)

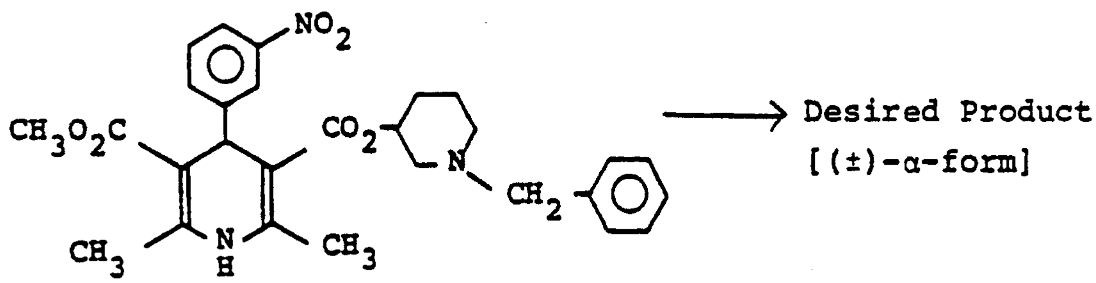


(I){Diastereomer [(±)-α-form, (±)-β-form]}

(wherein $R_1$ and $R_2$ have the same significances as defined above.)

As ammonium salts used in place of ammonia, ammonium acetate or ammonium carbonate are preferred.

Compound VII, IX and ammonia are in the molar ratio of 1.0 : 0.8 : 0.8 - 1.0 : 4.0 : 4.0, preferably, 1.0 : 0.9 : 0.9 - 1.0 : 1.5 : 1.5.

The reaction is carried out in the presence or absence of alcohols such as methanol, ethanol and isopropanol; aromatic hydrocarbons such as benzene and toluene; halogenated hydrocarbons such as chloroform, carbon tetrachloride and chlorobenzene; ethers such as tetrahydrofuran, dioxan and dimethoxyethane; aprotic polar solvents such as acetonitrile and N,N-dimethylformamide; or water at room temperature to 150° C, preferably 30° C to 100° C.

Process 5:

[a process in accordance with the process described in H. Herbert Fox et al., J. Org. Chem., 16, 1259 (1951)]

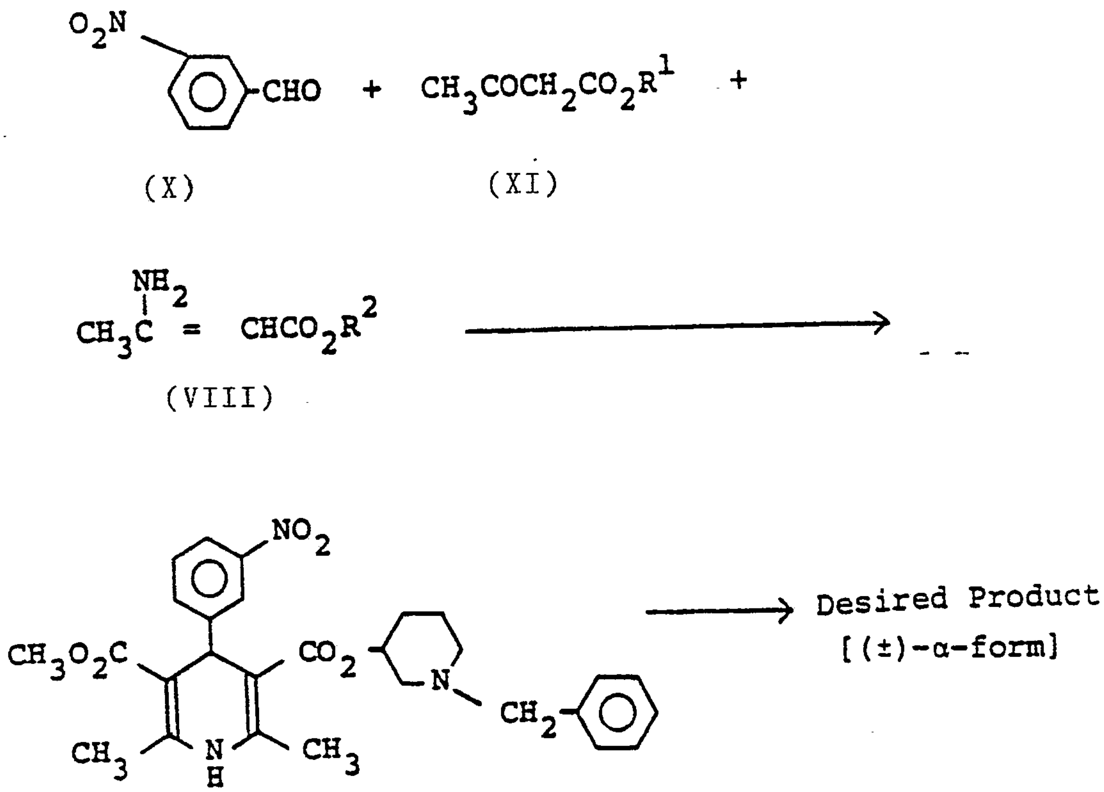


(X) (XI) (VIII)

(I){Diastereomer [(±)-α-form, (±)-β-form]}

wherein $R_1$ and $R_2$ have the same significances as defined above.

Compounds X, XI and VIII are in the molar ratio - of 1.0 : 0.8 : 0.8 - 1.0 : 4.0 : 4.0. preferably 1.0 : 0.9 : 0.9 - 1.0 : 1.5 : 1.5.

The reaction is carried out in the presence or absence of alcohols such as methanol, ethanol and isopropanol; aromatic hydrocarbons such as benzene and toluene; halogenated hydrocarbons such as chloroform and carbon tetrachloride; ethers such as tetrahydrofuran, dioxan and dimethoxyethane; aprotic polar solvents such as acetonitrile and N,N-dimethylformamide; or water at room temperature to 150 °C, preferably 30 °C to 100 °C.

Compounds II to III and VI to XI which are employed as starting materials in the processes of the present invention are either known compounds or compounds obtained in a conventional manner.

| | | |
|---|---|---|
| Compound II | : | T. Shibanuma et al., Chem., Pharm. Bull., 28, 2809 (1980) |
| Compound III | : | J.H. Biel et al., J. Org. Chem., 26, 4096 (1961) |
| Compound VII | : | G. Jones, The Knoevenagel condensation, Org. Reaction, 15, 204 (1967) |
| Compound VIII | : | S.A. Glickmann et al., J. Am. Chem. Soc., 67, 1017 (1945) |
| Compounds IX, XI | : | A.B. Boese, Ind. Eng. Chem., 32, 16 (1940) |
| Compounds VI, X | : | easily accessible as being commercially available |

In the reaction mixtures obtained in Processes 1 to 5, a compound [(±)-α-form], a melting point of the hydrochloride of which is 196°C to 202°C and a compound [(±)-β-form], a melting point of the hydrochloride of which is 236°C to 242°C are present. These are in relation of diastereomers, and are distinguished from each other by thin layer chromatography (TLC plate, Merck, Art 11798; developing solvent, dichloromethane : ethyl acetate : triethyl amine = 50 : 50 : 1 v/v) , wherein a compound showing a high Rf value is (±)-α-form. Isolation of the desired (±)-α-form from the mixture containing (±)-α-form and (±)-β-form is, for example, conducted by the following procedures.

After subjecting the reaction mixture to conventional operations such as extraction, concentration and column chromatography, hydrogen chloride gas or hydrogen chloride dissolved in a suitable solvent is added to the reaction mixture to form the hydrochloride (in case that the halogen of the halogenating reagent is chlorine in Process , the diastereomer represented by formula I is formed as the hydrochloride so that this operation is not necessarily required by applying a specified isolation method). After removing the solvent if necessary, fractional crystallization is carried out from a suitable single or mixed solvent, whereby (±)-α-form is crystallized out and (±)-β-form remains in the solution.

As single or mixed solvents suitable for the fractional crystallization, there are mentioned ethanol, chloroform, ethanol-acetone, chloroform-acetone, chloroform-ether or chloroform-ethyl acetate. The mixed solvents of ethanol-acetone and chloroform-acetone are particularly preferred.

In Compound V which is a starting material in Process 2, (±)-α'-form (a compound which becomes (±)-α-form when diastereomer I is prepared in accordance with Process 2) and (±)-β'-form (a compound which becomes (±)-β-form when diastereomer I is prepared in accordance with Process 2) are present, which are in relation of diastereomers, as in the case of diastereomers represented by formula I. Accordingly, when only (±)-α'-form of the starting Compound V is selectively used, the diastereomer obtained in Process 2 is only (±)-α-form so that operation for isolating (±)-α-form from (±)-β-form is unnecessary and (±)-α-form of diastereomer I is obtained in conventional operations such as extraction, concentration, column chromatography and crystallization.

As salts of the compound, there are mentioned inorganic salts such as hydrochloride, hydrobromide, phosphate and sulfate, and organic acid salts such as formate, acetate, fumarate, maleate and malate.

Next, the processes for producing (+)-α-form are shown below.

Process 6:

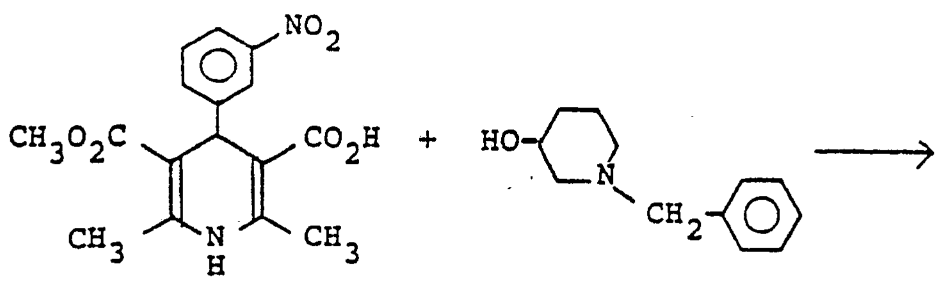

(XIII) (XIV)

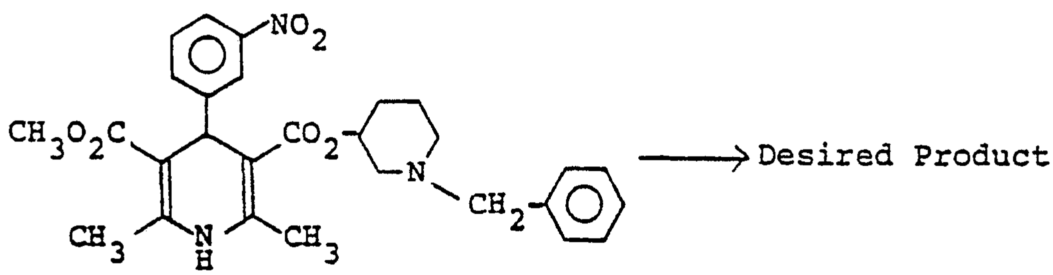


(XII)

An optically active form having an optical rotation degree of (-) or a racemate of the compound represented by the formula XIII (hereinafter referred to as Compound XIII; compounds of formulae XII and XIV are similarly referred to) is suitable as a starting material, and similarly an optically active form having an optical rotation degree of (+) or a racemate of Compound XIV is preferred as a starting material.

Esterification of Compounds XIII and XIV is carried out in a conventional manner, e.g.

a) a process which comprises converting Compound XIII into the acid halide using a halogenating reagent and then reacting the acid halide with Compound XIV;

b) a process which comprises reacting Compound XIII with Compound XIV in the presence of the condensing agent such as N,N'-dicyclohexylcarbodiimide; and the process a) using an inexpensive halogenating reagent (for example, thionyl chloride, phosphorus trichloride, phosphorus pentachloride, phosphorus oxychloride and phosphorus tribromide) is particularly advantageous.

The process a) is explained in further detail.

The process in which the halogenating reagent is thionyl chloride, is illustrated by the following equations:

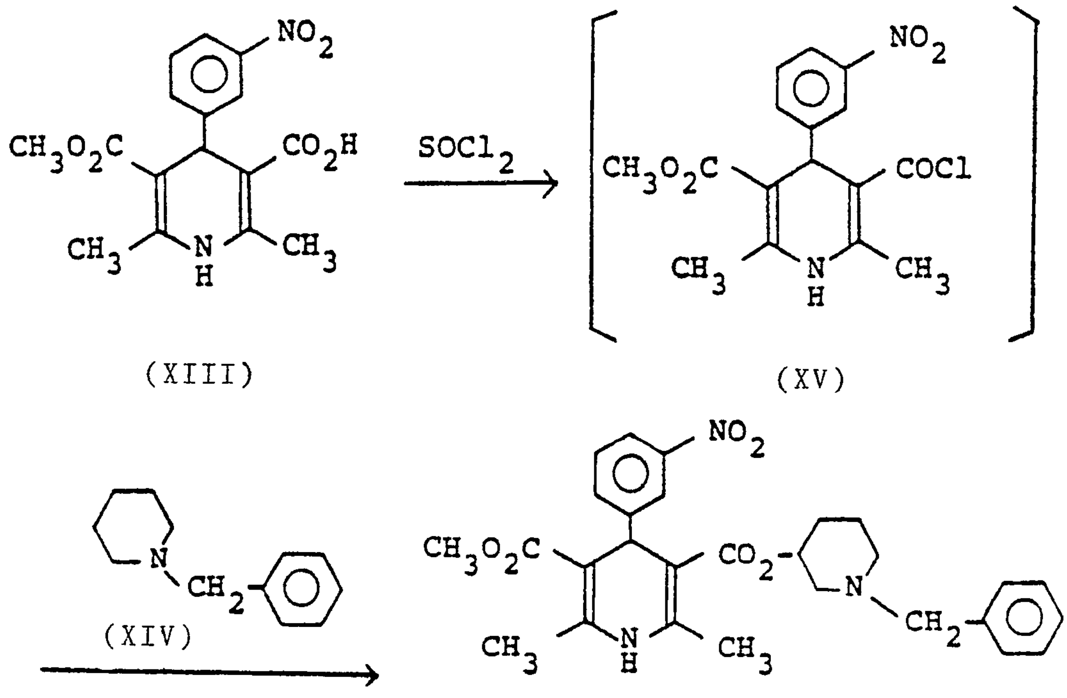


(XIII) (XV) (XIV)

(XII)

The reaction is carried out in the presence or absence of halogenated hydrocarbons such as dichloromethane, chloroform, carbon tetrachloride and chlorobenzene; aromatic hydrocarbons such as benzene and toluene; ethers such as tetrahydrofuran and dioxan; aprotic polar solvents such as acetonitrile, N,N-dimethylformamide and hexamethylphosphoric triamide; amines such as pyridine and triethyl amine. The reaction is preferably carried out using thionyl chloride as the halogenating reagent in the presence of N,N-dimethylformamide or hexamethylphosphoric triamide as a reaction solvent alone or using the aforesaid solvents in combination.

The molar ratio of Compound XIII to thionyl chloride ranges from 1.0 : 0.8 - 1.0 : 2.0, preferably 1.0 : 0.9 - 1.0 : 1.2.

The molar ratio of thionyl chloride to N,N-dimethylformamide or hexamethylphosphoric triamide is 1 : 1 - 1 : 100, preferably 1 : 5 - 1 : 50.

The reaction is carried out at temperatures of -70° C to 100° C, preferably -20° C to 50° C.

Then, the resulting Compound XV (which may not be isolated) is reacted with Compound XIV to thereby obtain Compound XII.

As the solvent, the same solvent as employed for preparation of Compound XV from Compound XIII is employed.

The molar ratio of Compound XIII to Compound XIV is in the range of 1.0 : 0.8 - 1.0 : 2.0, preferably 1.0 : 0.9 - 1.0 : 1.2 and the reaction temperature is -70° C to 100° C, preferably -20° C to 50° C.

Compound XIII and Compound XIV which are employed as starting materials are known compounds. Furthermore, optically active Compound XIV can also be readily produced from known optically active Compound XVI by the following reaction.

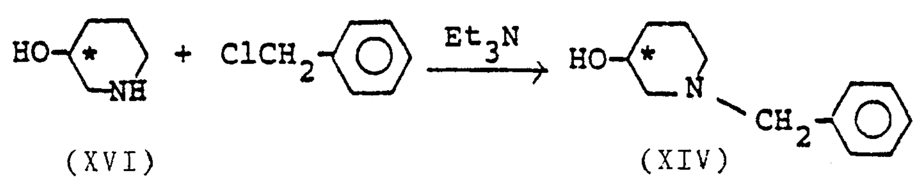


(XVI) (XIV)

(* represents an asymmetric carbon atom)

Compound XIII : T. Shibanuma et al., Chem. Pharm. Bull., 28, 2809 (1980)

Compound XVI : H. Sievertsson et al., J. Med. Chem., 15, 1085 (1972)

The desired product, (+)-α-form may be isolated from the resulting reaction mixture as follows.

In case that Compound XIII and Compound XIV are both optically active substances (Compound XIII is (-) form and Compound XIV is (+) form), the product is principally only (+)-α-form; therefore, the desired product can be isolated by procedures such as extraction and concentration, and if necessary, column chromatography may also be carried out.

In case that either Compound XIII or Compound XIV is a racemate, the desired product can be isolated by column chromatography in addition to extraction or concentration, since (+)-β-form or (-)-β-form is contained in the reaction mixture, in addition to the desired (+)-α-form. Furthermore, if deemed necessary, the isolated free product is reacted with inorganic acids such as hydrochloric acid, hydrobromic acid, phosphoric acid and sulfuric acid, or organic acids such as formic acid, acetic acid, fumaric acid, maleic acid, malic acid and tartaric acid to prepare salts thereof.

The hypotensive effect of (±)-α-form is explained below.

Testing Method

Mongrel adult dogs (8 to 15 kg) were anesthesized by the intravenous administration of 30 mg/kg of sodium pentobarbital. Each cannula was inserted into the left femoral artery, and the blood pressure was measured with a pressure transducer (Nippon Koden) and recorded on a polygraph.

The results are shown in Table 1.

**Table 1**

| Compound | Dose (µg/kg) | Number of Animals | | Blood Pressure before Administration (mm/Hg) | Blood Pressure before Administration 0.5 min | 1 min | 3 min | - Blood Pressure after Administration 10 min | 30 min | 60 min | 90 min |
|---|---|---|---|---|---|---|---|---|---|---|---|
| (±)-α-Form | 10 | 5 | Systolic blood pressure | 151.0±37 | 2±2 | 8±2 | 12±3 | 2±3 | 4±3 | 8±4 | 13±5 |
| | | | Diastolic blood pressure | 94.3±25 | 4±1 | 14±5 | 30±7 | 24±7 | 28±8 | 28±9 | 30±8 |
| (±)-β-Form as a control | 10 | 3 | Systolic blood pressure | 147 ± 20 | 3±1 | 4±1 | 3±2 | 1±2 | -2±1 | - | - |
| | | | Diastolic blood pressure | 103 ± 18 | 2±2 | 3±2 | 2±1 | 0±1 | -1±2 | - | - |

As shown in Table 1, (±)-α-form manifested a significant hypotensive effect one minute after the administration, which effect lasted for 90 minutes or longer.

Testing Method

The left renal artery of each mongrel dog (9 - 18 kg) was constricted under anesthetization with thiopental sodium to prepare renal hypertensive dogs. The blood pressure under nonanesthetic condition was hematoscopically measured via a polyethylene cannula (fixed to the back of the neck site) inserted into the descending aorta through the left corotid artery. The drug, suspended in a 0.3% aqueous carboxymethyl cellulose solution, was orally administered at a dose of 0.5 ml per kg - body weight using a tube for oral administration. The results are shown in Table 2.

Table 2

| Time (hr) | Dose (mg/kg) | Number of Animal | Systolic Blood Pressure (mmHg) | Diastolic Blood Pressure (mmHg) |
|---|---|---|---|---|
| 0 | | | 174.5 ± 8.4 | 100.0 ± 3.1 |
| 0.5 | | | 175.0 ± 8.5 | 96.0 ± 2.5 |
| 1 | | | 162.8 ± 7.0 | 88.5 ± 4.6 |
| 2 | | | 166.0 ± 6.5 | 91.5 ± 3.4 |
| 3 | 0.1 | 4 | 169.8 ± 7.7 | 94.8 ± 3.1 |
| 4 | | | 170.3 ± 7.1 | 96.8 ± 3.5 |
| 5 | | | 168.8 ± 6.6 | 97.5 ± 3.8 |
| 6 | | | 166.3 ± 7.1 | 98.3 ± 3.8 |
| 7 | | | 172.3 ± 6.5 | 99.0 ± 4.7 |
| 0 | | | 173.4 ± 8.1 | 99.6 ± 6.8 |
| 0.5 | | | 162.0 ± 13.2 | 87.0 ± 8.8 |
| 1 | | | 138.6 ± 15.6 | 76.0 ± 11.3 |
| 2 | | | 137.2 ± 13.8 | 74.6 ± 11.5 |
| 3 | 0.3 | 5 | 138.8 ± 10.2 | 78.6 ± 8.6 |
| 4 | | | 136.0 ± 10.7 | 79.0 ± 7.6 |
| 5 | | | 141.4 ± 10.7 | 80.8 ± 7.5 |
| 6 | | | 145.4 ± 9.2 | 83.0 ± 7.7 |
| 7 | | | 147.2 ± 9.3 | 86.2 ± 7.7 |
| 0 | | | 160.6 ± 5.9 | 96.8 ± 5.6 |
| 0.5 | | | 139.0 ± 14.9 | 76.8 ± 10.5 |
| 1 | | | 128.4 ± 11.3 | 62.6 ± 9.8 |
| 2 | | | 120.4 ± 8.0 | 58.6 ± 7.7 |
| 3 | 1 | 5 | 123.4 ± 7.1 | 63.6 ± 9.3 |
| 4 | | | 125.4 ± 8.2 | 63.6 ± 9.9 |
| 5 | | | 129.0 ± 7.7 | 64.8 ± 9.7 |
| 6 | | | 129.6 ± 9.1 | 70.6 ± 10.4 |
| 7 | | | 135.8 ± 8.9 | 73.2 ± 10.6 |

Note: Drug Used: (±)-α-form

Next, comparative tests of the hypotensive effect and acute toxicity among [(+)-α-form], (-)-α-form and (±)-α-form are described below.

Hypotensive Effect:

1. Intravenous Administration to SHR (no anesthesia)

Spontaneously hypertensive rats (SHR) (Okamoto strain, aging 20 to 30 weeks, male) were anesthetized with sodium thiopental (50 mg/kg, ip). A cannula for measuring blood pressure was inserted into the descending aorta from the right femoral artery and a cannula for intravenous administration was inserted into the left carotid vein, and these cannulas were fixed on the back of the neck, respectively. Change in blood pressure by intravenous administration of each of drugs was measured the next day without any anesthesia. As the drugs, a solution of each of the drugs in polyethylene glycol 400 was intravenously administered at the dose of 0.1 ml/l00 g body weight of the rat.

The results are shown in Table 3.

Table 3

| Drug | Dose Administered (μg/kg i.v.) | Number of Animal | Maximum Decrease in Mean Blood Pressure (mmHg) |
|---|---|---|---|
| (+)-α form | 1 | 5 | 42.2 ± 11.5 |
| (+)-α form | 10 | 5 | 67.4 ± 4.7 |
| (-)-α form | 10 | 5 | 23.4 ± 7.9 |
| (-)-α form | 100 | 5 | 27.3 ± 6.1 |
| (±)-α form | 1 | 5 | 23.1 ± 6.4 |
| (±)-α form | 10 | 5 | 45.6 ± 6.5 |

2. Oral Administration to SHR [no anesthesis)

Change in systolic blood pressure when drugs were orally administered to spontaneously hypertensive rats (SHR) (Okamoto Strain, aging 15 to 26 weeks, male) was measured by the plethysmographic tail method (Ueda Seisakusho, USM-105R).

Drugs were orally administered at the dose of 0.5 ml/100 g body weight of the rat in the form of a suspension in 0.3% carboxymethyl cellulose, using a gastric tube.

The results are shown in Table 4.

Table 4

| Drug | Dose Administered (mg/kg p.o.) | Number of Animal | Maximum Decrease in Blood Pressure (mmHg) |
|---|---|---|---|
| (+)-α form | 1 | 8 | 56.3 ± 13.0 |
| (-)-α form | 3 | 4 | 22.5 ± 9.6 |
| (-)-α form | 10 | 4 | 30.0 ± 5.3 |
| (±)-α form | 1 | 8 | 35.6 ± 6.3 |

Acute Toxicity:

Drugs were orally administered to mice (ddY type, body weight of 20 to 24 g, male) and the survival rate 72 hours after the administration was studied. A suspension of each of the drugs in water having added a small quantity of Tween 80 thereto was orally given at the dose of 0.1 ml/10 g mouse body weight, using a gastric tube.

| | Acute Toxicity $LD_{50}$ (mg/kg.po) |
|---|---|
| (+)-α-form | 95 |
| (-)-α-form | 918 |
| (±)-α-form | 218 |

The compounds of the present invention, in consideration of their pharmaceutical effect, may be employed in various pharmaceutical forms for the intended administration, and in particular, they are preferably employed in oral forms such as tablets and powders.

In the case of tablets, the compound of the present invention may be contained in an amount of 1 - 30% (W/W) per tablet. As other components (carriers), commonly employed excipients, disintegrators, lubricants, binders or coating agents may be employed.

For example, there may be mentioned excipients such as glucose and lactose, disintegrators such as starch and calcium carboxymethyl cellulose, lubricants such as magnesium stearate and talc, binders such as simple syrup, polyvinyl alcohol, gelatin and hydroxypropyl cellulose, and coating agents such as dispersants (e.g. methyl cellulose and ethyl cellulose) and plasticisers (e.g. glycerin and polyethylene glycol). Microcrystalline cellulose partakes of the properties of disintegrators of binders and of excipients.

In the case of powders, the compound of the present invention may be contained in an amount of 1 - 20% (W/W). As the carriers, excipients such as glucose and lactose and binders such as hydroxypropyl cellulose may be employed.

The $LD_{50}$ or (±)-α-form in the oral administration in male rats is 127 mg/kg. The dose is preferably in the range of 1 - 100 mg per day for a human adult weighing ca. - 60 kg.

Also, the $LD_{50}$ of (+)-α-form in the oral administration in male rats is 95 mg/kg. The dose is preferably in the range of 1 - 50 mg per day for a human adult weighing ca. 60 kg.

The present invention is more particularly discribed by the following examples and reference examples.

Example 1

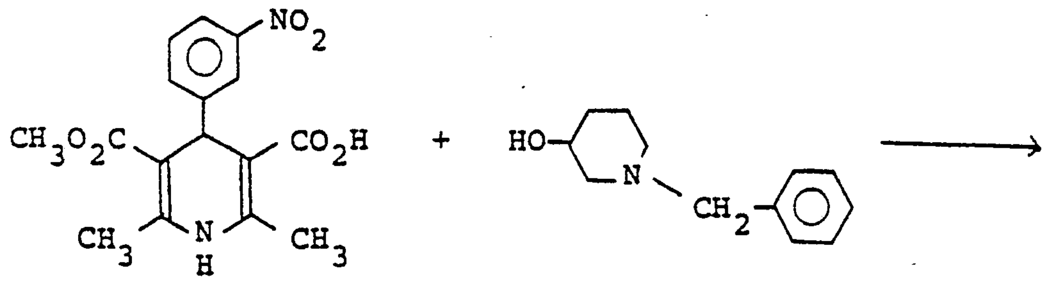

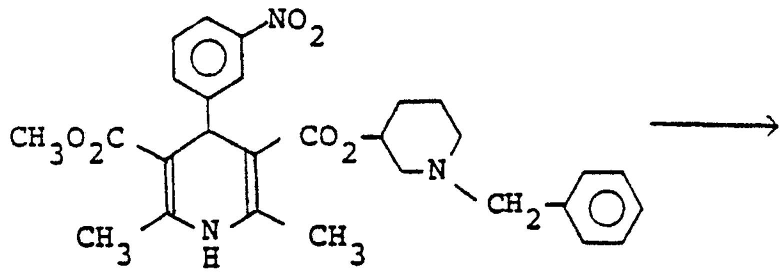

Desired Product
[(±)-α-form]

Diastereomer [(±)-α-form, (±)-β-form]

In this example, 10.00 g of 2,6-dimethyl-4-(3-nitrophenyl)-1,4-dihydropyridine-3,5-dicarboxylic acid monomethyl ester was suspended in 70 ml of a mixed solvent of dichloromethane and N,N-dimethylformamide (4 : 1 v/v) and 2.43 ml of thionyl chloride was added to the suspension under ice cooling. After stirring for one hour under ice cooling, 6.33 g of 1-benzyl-3-hydroxypiperidine was added thereto followed by further stirring under ice cooling for 2.5 hours. The reaction mixture was washed with 100 ml of water and then with 100 ml of brine. After drying the dichlorometahen layer over anhydrous sodium sulfate, the layer was concentrated under reduced pressure. Thereafter, 100 ml of acetone and 8 ml of ethanol were added to the concentrate to obtain 7.57 g of yellow substance of 2,6-dimethyl-4-(3-nitrophenyl)-1,4-dihydropyridine-3,5-dicarboxylic acid-3-(1-benzyl-3-piperidyl) ester-5-methyl ester hydrochloride [(±)-α-form]. The melting point was 197 °C to 198 °C (ethanol). This compound shows a single spot on TLC (Merck, Art 11798; developing solvent, dichloromethane : ethyl acetate : triethyl amine = 50 : 50 : 1 v/v).

From the mother liquor of the crystals, 5.21 g of (±)-β-form was obtained. The melting point was 239 °C to 240 °C (ethanol-methanol). Physical properties of (±)-α-form are shown below.

| | |
|---|---|
| IR (KBr, $cm^{-1}$): | 1680, 1525, 1345 |
| NMR (DMSO-$d_6$, δ): | 1.3 - 2.2(4H, broad), 2.33(6H, s), 2.7 - 3.4(4H, broad), 3.57(3H, s), 4.40(2H, s), 4.98 (1H, s), 5.20(1H, broad), 7.3 - 8.2(9H, m), 9.47(1H, broad) |

Elemental Analysis (as $C_{28}H_{32}ClN_3O_6$):

| | C | H | N |
|---|---|---|---|
| Found (%) | 62.16 | 6.01 | 7.76 |
| Calcd. (%) | 62.04 | 5.96 | 7.75 |

Example 2

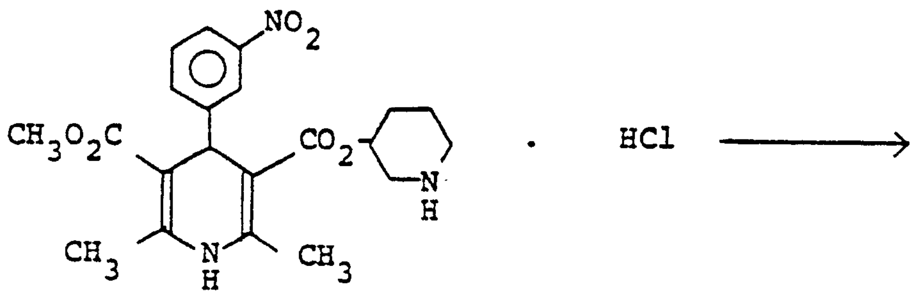


[(±)-α'-form]

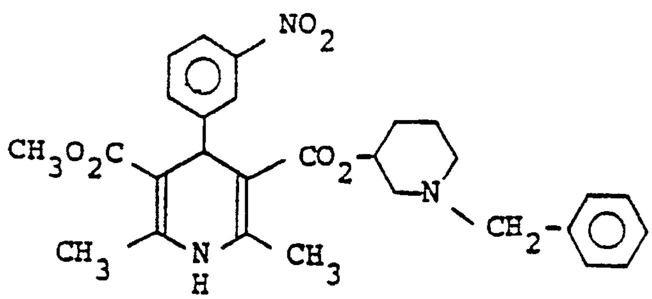

Desired Product [(±)-α-form]

In this example, 1.35 g of 2,6-dimethyl-4-(3-nitrophenyl)-1,4-dihydropyridine-3,5-dicarboxylic acid-3-methyl-5-(3-piperidyl) ester hydrochloride [(±)-α'-form] having a melting point of 236° C to 238° C obtained in Reference Example 1, 0.52 g of benzyl chloride and 0.62 g of triethyl amine were added to 20 ml of THF, and stirring was effected for 24 hours under reflux. The reaction mixture was poured into 50 ml of water. The mixture was extracted twice with 30 ml of ethyl acetate. The ethyl acetate layers were combined and 10 ml of 4N hdyrochloric acid was added thereto. After vigorously stirring the mixture for one hour, the ethyl acetate layer was washed with 20 ml of brine and then with 10 ml of water. After drying over anhydrous sodium sulfate, the mixture was concentrated under reduced pressure. To the concentrate was added 10 ml of a mixed solvent of chloroform and acetone (1 : 9 v/v) and the mixture was stirred to obtain 1-22 9 of yellow substance of 2,6-dimethyl-4-(3-nitrophenyl)-1,4-dihydropyridine-3,5-dicarboxylic acid-3-(1-benzyl-3-piperidyl) ester-5-methyl ester hydrochloride [(±)-α-form]. The melting point was 197° C to 198° C (ethanol). TLC, IR and NMR of the compound obtained in this example were identical with those of (±)-α-form obtained in Example 1.

Example 3

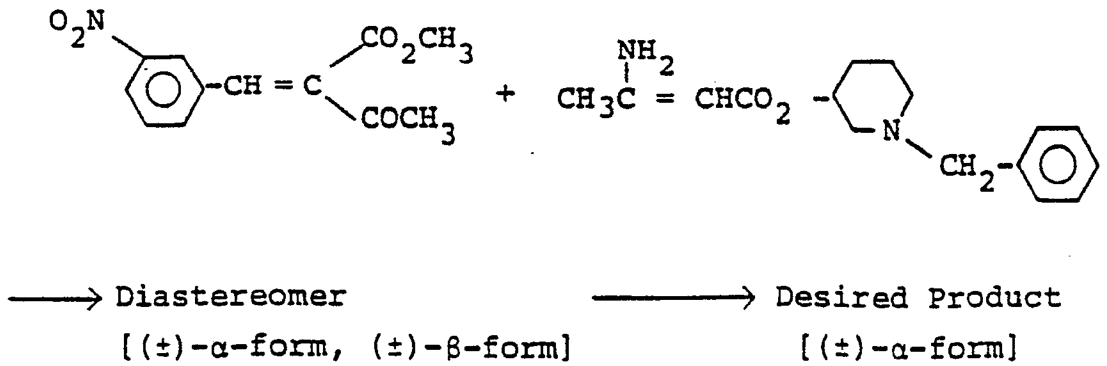


In this example, 2.49 g of methyl 3-nitrobenzylidene acetoacetate and 2.74 g of 3-aminocrotonic acid-1-benzyl-3-piperidyl ester were added to 10 ml of methanol, and stirring was effected for 10 hours under reflux. The reaction mixture was concentrated under reduced pressure and fractions containing the desired compound [(±)-α-form] were separated therefrom by silica gel column chromatography (eluent, chloroform : methanol = 20 : 1 v/v). After concentrating the fractions containing the desired compound, the concentrate was dissolved in 40 ml of chloroform, and 10 ml of 4N hydrochloric acid was added thereto. The mixture was vigorously stirred. Thereafter, the chloroform layer was washed twice with 20 ml of water, dried over anhydrous sodium sulfate and reconcentrated. A mixed solvent of 3 ml of ethanol and 27 ml of acetone was added to the concentrate, and the mixture was stirred to obtain 1.61 g of yellow substance of 2,6-dimethyl-4-(3-nitrophenyl)-1,4-dihydropyridine-3,5-dicarboxylic acid-3-(1-benzyl-3-piperidyl) ester-5-methyl ester hydrochloride [(±)-α-form]. The melting point was 197° C to 198° C (ethanol). TLC, IR and NMR spectra of the compound obtained in this example were identical with those of (±)-α-form obtained in Example 1.

Example 4

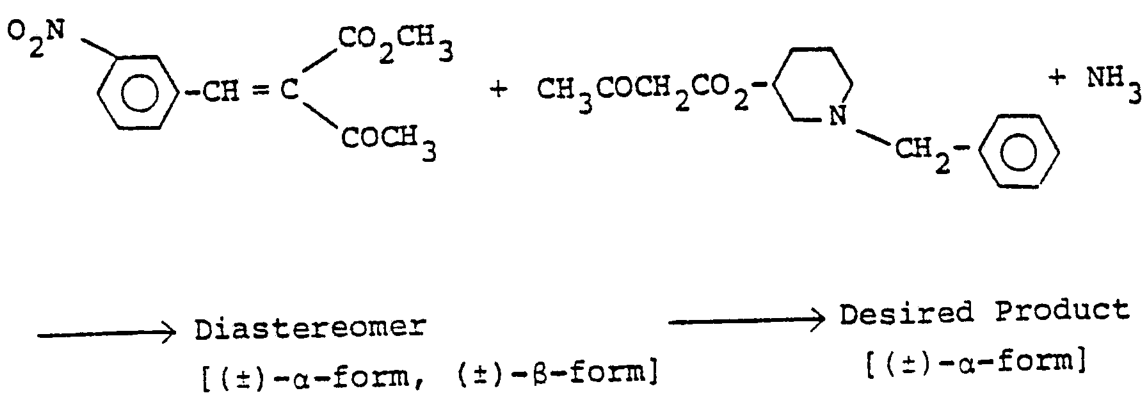


In this example, 4.98 g of methyl 3-nitrobenzylidene acetoacetate, 6.05 g of acetoacetic acid-1-benzyl-3-piperidyl ester and 1.76 g of 29% aqueous ammonia solution were added to 20 ml of ethanol, and stirring was effected for 12 hours under reflux. The reaction mixture was treated in a similar manner to Example 3 to obtain 3.02 g of 2,6-dimethyl-4-(3-nitrophenyl)-1,4-dihydropyridine-3,5-dicarboxylic acid-3-(1-benzyl-3-piperidyl) ester-5-methyl ester hydrochloride [(±)-α-form]. The melting point was 196.5° C to 198° C (ethanol). TLC, IR and NMR spectra of the compound obtained in this example were identical with those of (±)-α-form obtained in Example 1.

Example 5

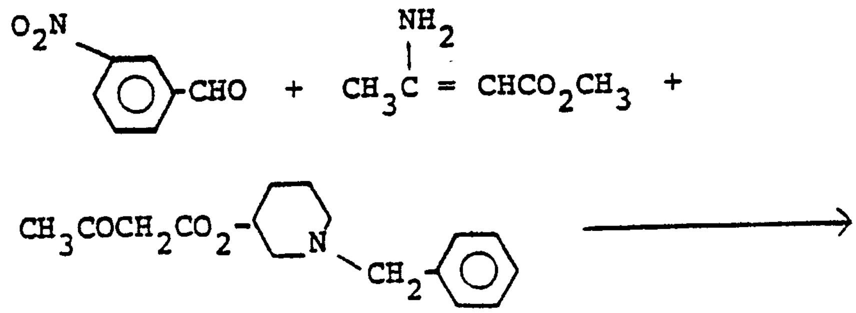

Diastereomer [(±)-α-form, (±)-β-form]

Desired Product [(±)-α-form]

In this example, 3.02 g of m-nitrobenzaldehyde, 2.53 g of methyl β-aminocrotonate and 5.50 g of acetoacetic acid-1-benzyl-3-piperidyl ester were added to 10 ml of THF, and stirring was effected for 19 hours under reflux. The reaction mixture was concentrated under reduced pressure, and fractions containing the desired compound were separated from the concentrate by silica gel column chromatography (eluent, chloroform : acetone = 9 : 1 v/v), and concentrated. The concentrate was dissolved in 70 ml of acetone and adjusted to pH 1 with ether saturated with hydrogen chloride. Further, ether was added thereto to give 2.10 g of yellow crystals. The melting point was 176° C to 178° C. Recrystallization from acetone-ethanol (9 : 1 v/v) gave 0.93 g of 2,6-dimethyl-4-(3-nitrophenyl)-1,4-dihydropyridine-3,5-dicarboxylic acid-3-(1-benzyl-3-piperidyl) ester-5-methyl ester hydrochloride [(±)-α-form]. The melting point was 197° C to 198.5° C. TLC, IR and NMR spectra of the compound obtained in this example were identical with those of (±)-α-form obtained in Example 1.

Example 6

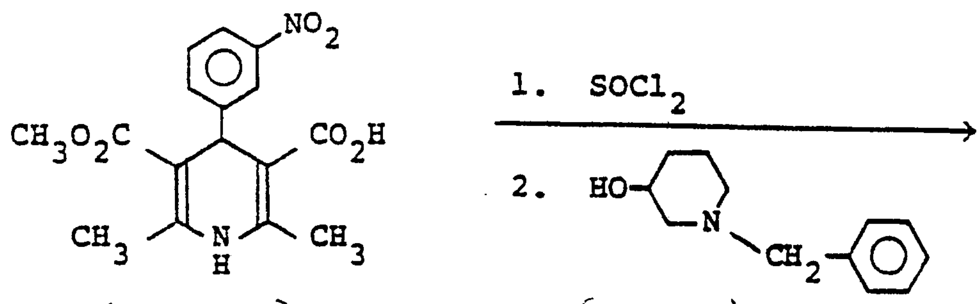


((-)-XIII) ((+)-XIV)

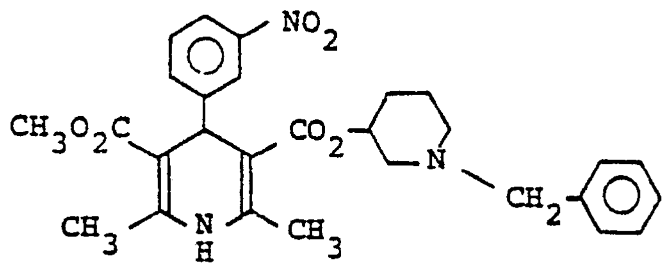

(XII) [(+)-α-form]

In 17 ml of a mixed solvent of dichloromethane and N,N-dimethylformamide (4 : 1 v/v), 2.50 g of (-)-2,6-dimethyl-4-(3-nitrophenyl)-1,4-dihydropyridine-3,5-dicarboxylic-acid monomethyl ester was suspended and to the suspension was added 0.57 ml of thionyl chloride under ice cooling. After stirring under ice cooling for one hour, 1.51 g of (+)-1-benzyl-3-hydroxypiperidine obtained in Reference Example 2 was added to the mixture and stirring was further continued under ice cooling for 2 hours. The reaction mixture was diluted with 25 ml of dichloromethane, and washed with 30 ml of water and then with 30 ml of brine. The dichloromethane layer was dried over anhydrous sodium sulfate and concentrated under reduced pressure. The concentrate was subjected to silica gel column chromatography (eluent, chloroform : ethyl acetate = 1 : 1 v/v) and fractions containing the desired product were concentrated. To the concentrate were added 30 ml of chloroform and 10 ml of 4N hydrochloric acid. After vigorously stirring the mixture, the chloroform layer was washed twice with 20 ml of water, then dried over anhydrous sodium sulfate, and concentrated to dryness to obtain 4.20 g of amorphous powder. The powders were dissolved in 15 ml of acetone and the solution was added to 200 ml of ether. The mixture was stirred to observe precipitates. The precipitates were filtered and dried to obtain 2.72 g of (+)-α-2,6-dimethyl-4-(3-nitrophenyl)-1,4-dihydropyridine-3,5-dicarboxylic acid-3-(1-benzyl-3-piperidyl) ester-5-methyl ester hydrochloride [(+)-α-form].

$[\alpha]_D^{21}$ + 113.6 (c = 0.50, acetone)

IR (KBr, $cm^{-1}$): 1680, 1525, 1350

NMR (DMSO-$d_6$, δ): 1.3 - 2.2(4H, broad), 2.33(6H, s), 2.7 - 3.4(4H, broad), 3.57(3H, s), 4.40(2H, s), 4.98 (1H, s) , 5.20(1H, broad) , 7.3 - 8.2(9H, m), 9.47(1H, broad)

Example 7

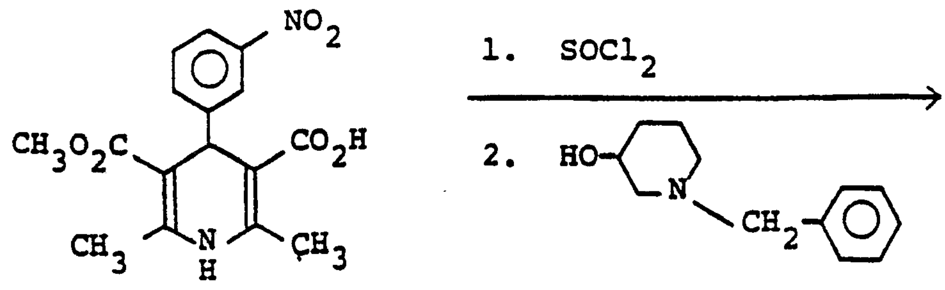


(XIII) ((+)-XIV)

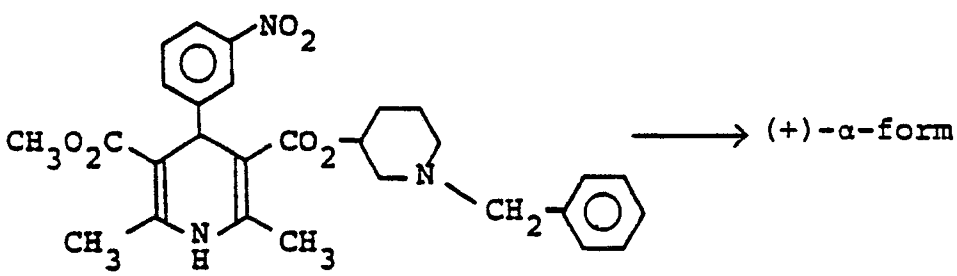


(XII) (mixture)

In 17 ml of a mixed solvent of dichloromethane and N,N-dimethylformamide (4 : 1 v/v), 2-50 g of 2,6-dimethyl-4-(3-nitrophenyl)-1,4-dihydropyridine-3,5-dicarboxylic acid monomethyl ester was suspended and to the suspension was added 0.57 ml of thionyl chloride under ice cooling. After stirring under ice cooling for one hour, 1.51 g of (+)-1-benzyl-3-hydroxypiperidine obtained in Reference Example 2 was added to the mixture and stirring was further continued under ice cooling for 2 hours and 40 minutes. The reaction mixture was diluted with 50 ml of dichloromethane and washed with 50 ml of 5% sodium hydrogen carbonate solution, 50 ml of water and then 50 ml of brine. The dichloromethane layer was dried over anhydrous sodium sulfate, and concentrated under reduced pressure. The concentrate was subjected to silica gel column chromatography (eluent, chloroform : ethyl acetate : triethyl amine = 50 : 50 : 1 v/v) to separate (+)-α-form from (+)-β-form. Fractions containing the desired (+)-α-form were concentrated and dissolved in 20 ml of acetone. To the solution was added 5 ml of 4N hydrochloric acid. After stirring the mixture, acetone was removed by distillation under reduced pressure. To the residue remained after the concentration was added 20 ml of chloroform. The chloroform layer was washed twice with 20 ml of water, then dried over anhydrous sodium sulfate, and concentrated to dryness to obtain 1.02 g of amorphous powders of (+)-α-2,6-dimethyl-4-(3-nitrophenyl)-1,4-dihydropyridine-3,5-dicarboxylic acid-3-(1-benzyl-3-piperidyl) ester-5-methyl ester hydrochloride [(+)-α-form]

$[\alpha]_D^{21}$ + 110.7 (c = 0.50, acetone)

IR and NMR spectra were identical with those of (+)-α-form obtained in Example 6.

Reference Example 1

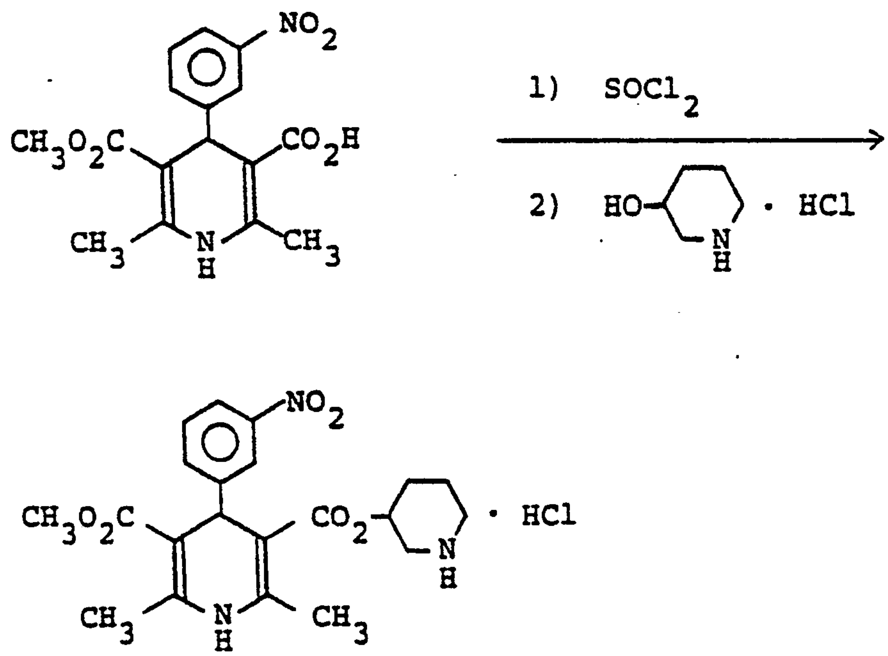


In this example, 3.31 g of 2,6-dimethyl-4-(3-nitrophenyl)-1,4-dihydropyridine-3,5-dicarboxylic acid monomethyl ester was suspended in 23 ml of a mixed solvent of dichloromethane and N,N-dimethylformamide (4 : 1 v/v), and further 1.32 g of thionyl chloride was added to the suspension under ice cooling. After stirring the mixture for one hour under ice cooling, 1.51 g of 3-hydroxypiperidine hydrochloride was added thereto and the mixture was further stirred for 1 hour and 20 minutes under ice cooling. After completion of the reaction, the reaction mixture was dissolved in 50 ml of chloroform and the solution was washed with 40 ml of water. Then, 40 ml of 1N sodium hydroxide was added and the mixture was vigorously stirred. The chloroform layer was separated out. After washing the chloroform layer twice with 20 ml of water, the chloroform layer was concentrated. The concentrate was dissolved in 20 ml of acetone and 2 ml of conc. hydrochloric acid was added to the solution. Stirring under ice cooling gave 2.07 g of 2,6-dimethyl-4-(3-nitrophenyl)-1,4-dihydropyridine-3,5-dicarboxylic acid-3-methyl-5-(3-piperidyl) ester hydrochloride [(±)-α'-form].

| | |
|---|---|
| Melting point: | 236 °C - 238 °C |
| IR (Nujol, $cm^{-1}$): | 1695, 1530, 1350 |
| NMR (DMSO-$d_6$, δ): | 1.4 - 2.1(4H, broad), 2.32(3H, s), 2.35(3H, s), 2.7 - 3.3(4H, broad), 3.56(3H, s), 4.7 - 5.2(1H, broad), 5.05(1H, s), 7.4 - 8.1(4H, m), 9.33(1H, s) |

Elemental Analysis (as $C_{21}H_{25}N_3O_6 \cdot HCl$)

| | C | H | N |
|---|---|---|---|
| Found (%) | 55.73 | 5.74 | 9.42 |
| Calcd. (%) | 55.81 | 5.80 | 9.30 |

Further from the mother liquor of the aforesaid crystals, 1.27 g of 2,6-dimethyl-4-(3-nitrophenyl)-1,4-dihydropyridine-3,5-dicarboxylic acid-3-methyl-5-(3-piperidyl) ester hydrochloride [(±)-β'-form] having a melting point of 248.5 °C was obtained.

Reference Example 2

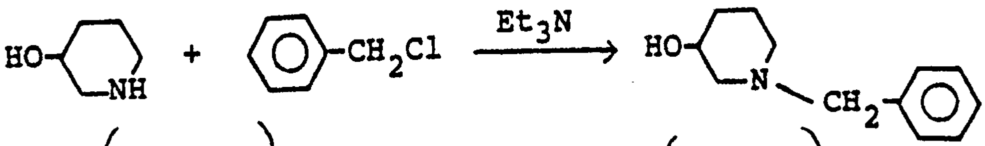


((-)-XVI) ((+)-XIV)

After stirring 8.53 g of (-)-3-hydroxypiperidine, 7.72 g of benzyl chloride and 6.17 g of triethyl amine in 70 ml of toluene for 5 hours under reflux, the mixture was filtered and the filtrate was concentrated under reduced pressure. The residue was distilled under reduced pressure to obtain 5.49 g of a distillate having a boiling point of 103.8° C/0.5 mmHg.

$[\alpha]_D^{23}$ + 11.9 (c = 2.14, methanol)

<u>Reference Example 3</u>

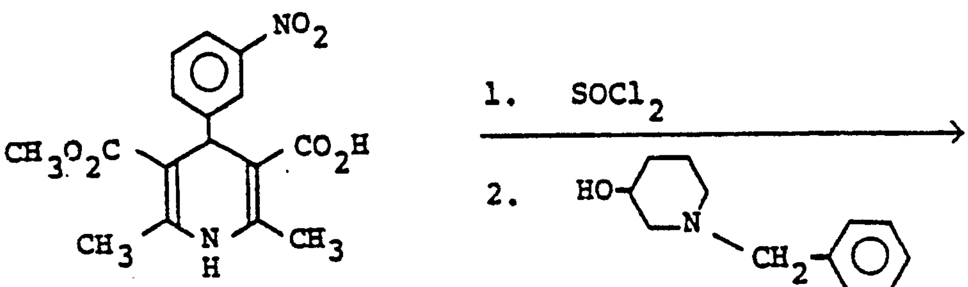


((+)-XIII) ((-)-XIV)

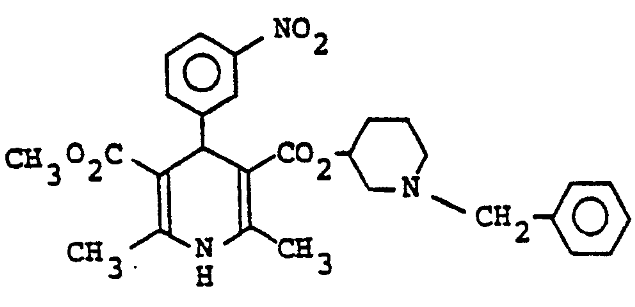

(XII)[(-)-α-form]

In a similar manner to Example 6, 3.16 g of amorphous powders of (-)-α-2,6-dimethyl-4-(3-nitrophenyl)-1,4-dihydropyridine-3,5-dicarboxylic acid-3-(1-benzyl-3-piperidyl) ester-5-methyl ester hydrochloride were obtained except that 2.50 g of (+)-2,6-dimethyl-4-(3-nitrophenyl)-1,4-dihydropyridine-3,5-dicarboxylic acid monomethyl ester and 1.51 g of (-)-1-benzyl-3-hydroxypiperidine were employed in place of (-)-2,6-dimethyl-4-(3-nitrophenyl)-1,4-dihydropyridine-3,5-dicarboxylic acid monomethyl ester and (+)-1-benzyl-3-hydroxypiperidine used in Example 6, respectively.

$[\alpha]_D^{22}$ - 110.2 (c = 0.24, acetone)

## Claims

1. (±)-2,6-Dimethyl-4-(3-nitrophenyl)-1,4-dihydropyridine-3,5-dicarboxylic acid-3-(1-benzyl-3-piperidyl) ester-5-methyl ester, the melting point of the hydrochloride of which is 196 °C to 202 °C or a salt thereof.

2. A process for producing a compound as defined in claim 1, which comprises the steps of reacting a compound represented by the formula II:

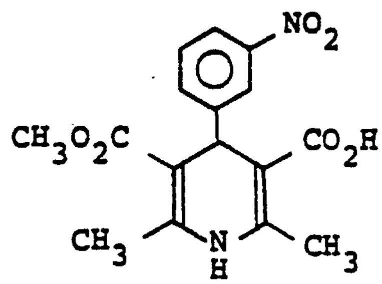

( II )

with a compound represented by the formula III:

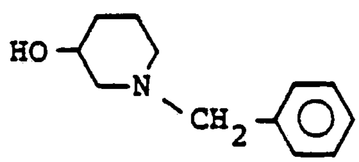

( III )

to give a diastereomer represented by the formula I:

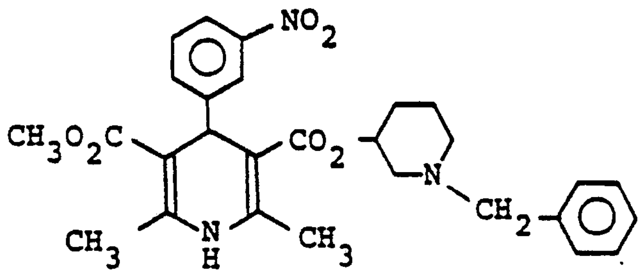

( I )

and subjecting said diastereomer to fractional crystallization.

3. A process for producing a compound as defined in claim 1, which comprises the steps of reacting a compound represented by the formula V:

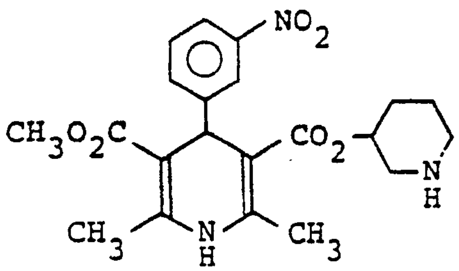

( V )

with a compound represented by the formula VI:

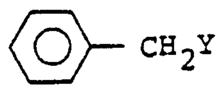

( VI )

(wherein Y represents a halogen atom)
to give a diastereomer represented by the formula I and subjecting said diastereomer to fractional crystallization.

4. A process for producing a compound as defined in claim 1, which comprises the steps of reacting a compound represented by the formula VII:

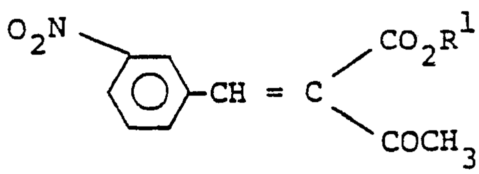

( VII )

with a compound represented by the formula VIII:

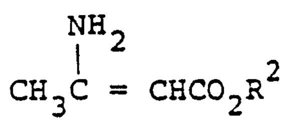

( VIII )

(wherein one of $R_1$ and $R_2$ represents a methyl group, and the other represents a benzylpiperidyl group) to give a diastereomer represented by the formula I and subjecting said diastereomer to fractional crystallization.

5. A process for producing a compound as defined in claim 1, which comprises the steps of reacting a compound represented by the formula VII:

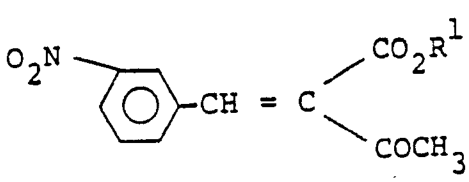

( VII )

with a compound represented by the formula IX:

$$CH_3COCH_2CO_2R^2 \qquad (IX)$$

(wherein $R_1$ and $R_2$ have the same significances as defined above) and with $NH_3$ (or ammonium salt) to give a diastereomer represented by the formula I and subjecting said diastereomer to fractional crystallization.

6. A process for producing a compound as defined in claim 1, which comprises the steps of reacting a compound represented by the formula X:

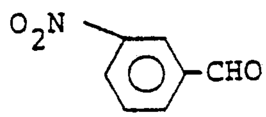

( X )

with a compound represented by the formula XI:

$$CH_3COCH_2CO_2R^1 \qquad (XI)$$

and with a compound represented by the formula VIII

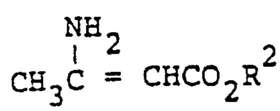

( VIII )

(wherein $R_1$ and $R_2$ have the same significances as defined above) to give a diastereomer represented by the formula I and subjecting said diastereomer to fractional crystallization.

7. An optical isomer having an optical rotation degree of (+) from among (±)-2,6-dimethyl-4-(3-nitrophenyl)-1,4-dihydropyridine-3,5-dicarboxylic acid-3-(1-benzyl-3-piperidyl) ester-5-methyl ester, the hydrochloride of which has melting points of 196° C to 202° C or a salt thereof.

8. A process for producing a compound as defined in claim 7, which comprises the steps of reacting a compound represented by the formula XIII:

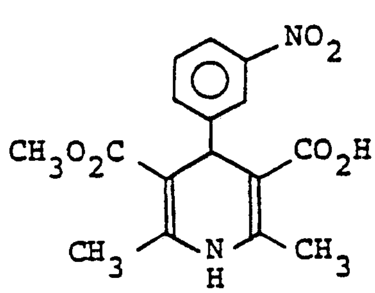

( XIII )

with a compound represented by the formula XIV:

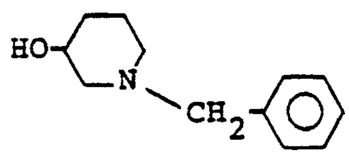

( XIV )

to give a compound represented by the formula XII:

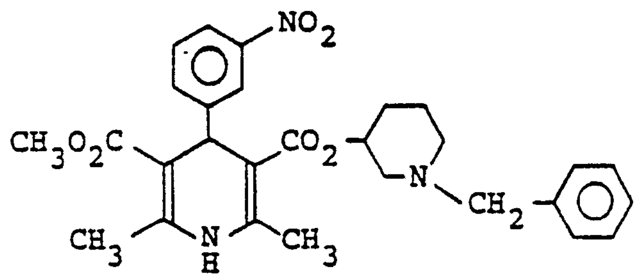

( XII )

and isolating a compound as defined in claim 7 from the reaction mixture in a conventional manner, in case that Compound XIII and Compound XIV are both optically active substances; or by column chromatography, in case that either Compound XIII or Compound XIV is a racemate.

**9.** A pharmaceutical composition comprising a pharmaceutical carrier and as active ingredient, an effective amount of a compound as defined in claim 1.

**10.** A pharmaceutical composition comprising a pharmaceutical carrier and as active ingredient, an effective amount of a compound as defined in claim 7.

CLAIMS FOR THE CONTRACTING STATE: AT

**1.** A process for producing a (±)-2,6-dimethyl-4-(3-nitrophenyl)-1,4-dihydropyridine-3,5-dicarboxylic acid-3-(1-benzyl-3-piperidyl) ester-5-methyl ester, the melting point of the hydrochloride of which is 196 °C to 202 °C or a salt thereof
which comprises the steps of reacting a compound represented by the formula II:

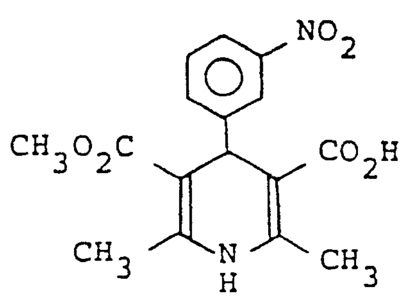

(II)

with a compound represented by the formula III:

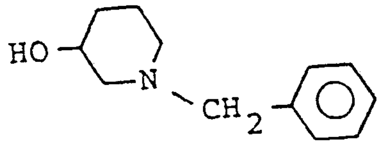

(III)

to give a diastereomer represented by the formula I:

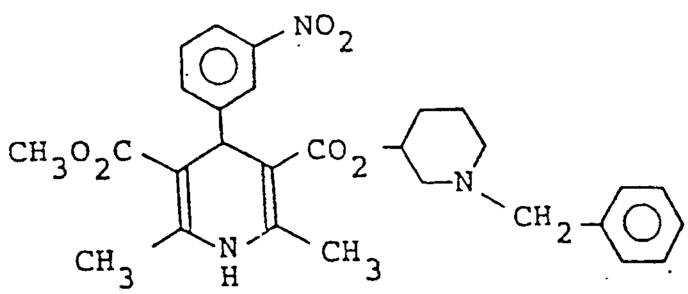

(I)

and subjecting said diastereomer to fractional crystallization.

2. A process for producing a (±)-2,6-dimethyl-4-(3-nitrophenyl)-1,4-dihydropyridine-3,5-dicarboxylic acid-3-(1-benzyl-3-piperidyl) ester-5-methyl ester, the melting point of the hydrochloride of which is 196°C to 202°C or a salt thereof
which comprises the steps of reacting a compound represented by the formula V:

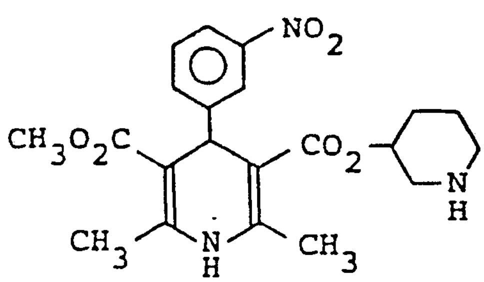

( V )

with a compound represented by the formula VI:

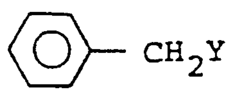

(VI)

(wherein Y represents a halogen atom)
to give a diastereomer represented by the formula I and subjecting said diastereomer to fractional crystallization.

3. A process for producing a

(±)-2,6-dimethyl-4-(3-nitrophenyl)-1,4-dihydropyridine-3,5-dicarboxylic acid-3-(1-benzyl-3-piperidyl) ester-5-methyl ester, the melting point of the hydrochloride of which is 196°C to 202°C or a salt thereof
which comprises the steps of reacting a compound represented by the formula VII:

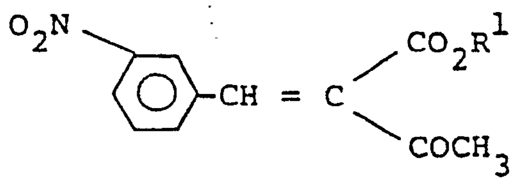

(VII)

with a compound represented by the formula VIII:

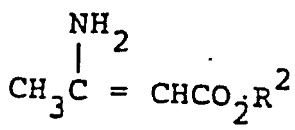

(VIII)

(wherein one of $R_1$ and $R_2$ represents a methyl group, and the other represents a benzylpiperidyl group) to give a diastereomer represented by the formula I:

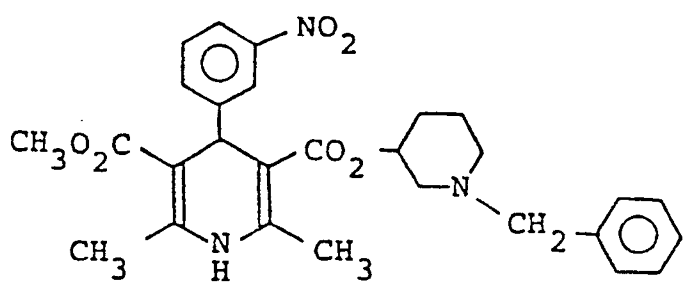

( I )

and subjecting said diastereomer to fractional crystallization.

4. A process for producing a
(±)-2,6-dimethyl-4-(3-nitrophenyl)-l,4-dihydropyridine-3,5-dicarboxylic acid-3-(1-benzyl-3-piperidyl) ester-5-methyl ester, the melting point of the hydrochloride of which is 196°C to 202°C or a salt thereof
which comprises the steps of reacting a compound represented by the formula VII:

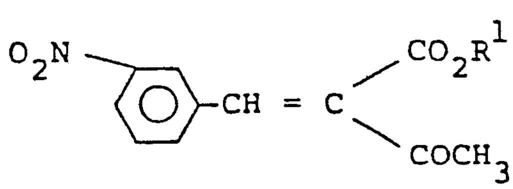

( VII )

with a compound represented by the formula IX:

$$CH_3COCH_2CO_2R^2 \qquad (IX)$$

(wherein $R_1$ and $R_2$ have the same significances as defined above) and with $NH_3$ (or ammonium salt) to give a diastereomer represented by the formula I

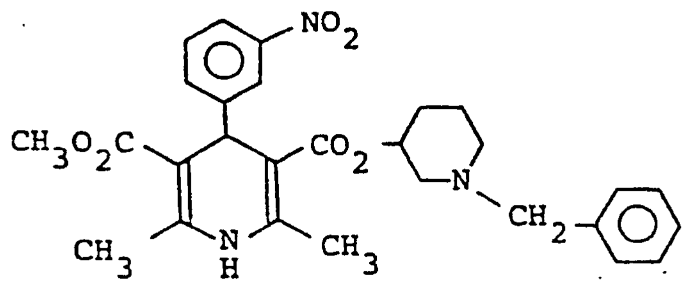

( I )

and subjecting said diastereomer to fractional crystallization.

**5.** A process for producing a (±)-2,6-dimethyl-4-(3-nitrophenyl)-1,4-dihydropyridine-3,5-dicarboxylic acid-3-(1-benzyl-3-piperidyl) ester-5-methyl ester, the melting point of the hydrochloride of which is 196°C to 202°C or a salt thereof,
which comprises the steps of reacting a compound represented by the formula X:

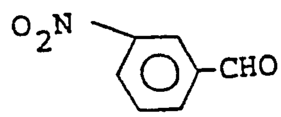

(X)

with a compound represented by the formula XI:

$$CH_3COCH_2CO_2R^1 \qquad (XI)$$

and with a compound represented by the formula VIII

$$\overset{NH_2}{CH_3C} = CHCO_2R^2 \qquad (VIII)$$

(wherein $R_1$ and $R_2$ have the same significances as defined above) to give a diastereomer represented by the formula I

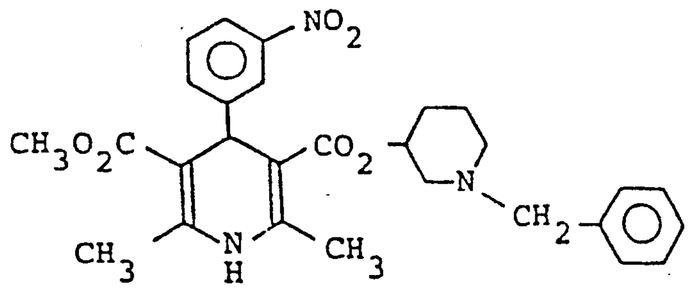

( I )

and subjecting said diastereomer to fractional crystallization

6. A process for producing
an optical isomer having an optical rotation degree of (+) from among (±)-2,6-dimethyl-4-(3-nitrophenyl)-1,4-dihydropyridine-3,5-dicarboxylic acid-3-(1-benzyl-3-piperidyl) ester-5-methyl ester, the hydrochloride of which has melting points of 196°C to 202°C or a salt thereof
which comprises the steps of reacting a compound ,represented by the formula XIII:

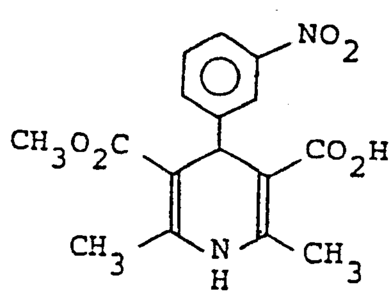

( XIII )

with a compound represented by the formula XIV:

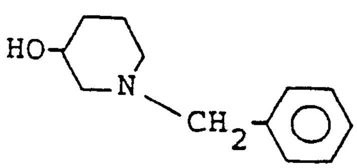

( XIV )

to give a compound represented by the formula XII:

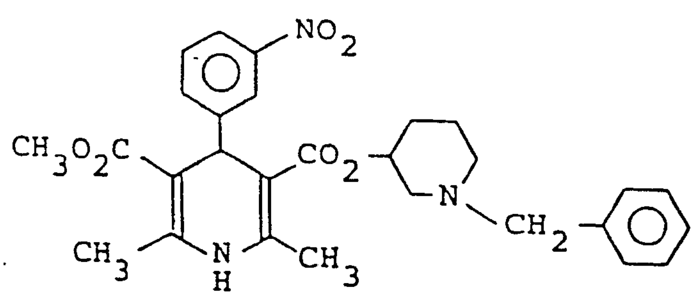

( XII )

and isolating said optical isomer having an optical rotation degree of (+) from the reaction mixture in a

conventional manner, in case ,that compound XIII and compound XIV are both optically active substances; or by column chromatography, in case that either compound XIII or compound XIV is a racemate.

7. Process for producing the desired (±)-α form of the hydrochloride of 2,6-dimethyl-4-(3-nitrophenyl)-1,4-dihydropyridine-3,5-dicarboxylic acid-3-(1-benzyl-3-piperidyl) ester-5-methyl ester having a melting point of 196° C to 202° C which comprises subjecting the reaction mixture obtained by a process according to anyone of claims 1 to 5 to extraction, concentration and/or column chromatography, then adding hydrogen chloride gas or hydrogen chloride salt in a suitable solvent to form the hydrochloride and after removing the solvent,if necessary,carrying out fractional crystallization from a suitable single or mixted solvent whereby (±)-α form is crystallized out and (±)-β form remains in the solution.

8. Process according to claim 7 wherein ethanol-acetone or chloroform-acetone is used as mixed solvents for the fractional crystallization.

9. Process for producing a pharmaceutical composition comprising the steps of producing a (±)-2,6-dimethyl-4-(3-nitrophenyl)-1,4-dihydropyridine-3,5-dicarboxylic acid-3-(1-benzyl-3-piperidyl) ester-5-methyl ester, the melting point of the hydrochloride of which is 196° C to 202° C or a salt thereof according to anyone of claims 1 to 7 and mixing an effective amount of said compound with a pharmaceutical carrier.

## Revendications

1. Ester 5-méthylique et 3-(1-benzyl-3-pipéridylique) de l'acide (±)-2,6-diméthyl-4-(3-nitrophényl)-1,4-dihydropyridine-3,5-dicarboxylique, dont le chlorhydrate a un point de fusion de 196 à 202° C, ou l'un de ses sels.

2. Procédé de préparation d'un composé tel que défini dans la revendication 1, qui comprend les étapes de réaction d'un composé représenté par la formule II :

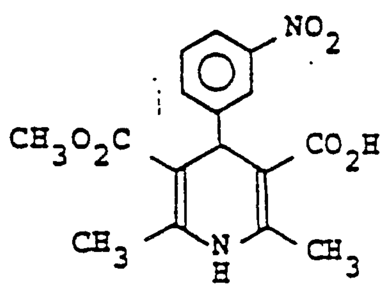

(II)

avec un composé représenté par la formule III :

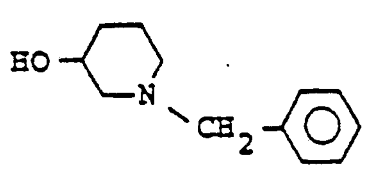

(III)

pour donner un diastéréoisomère représenté par la formule I :

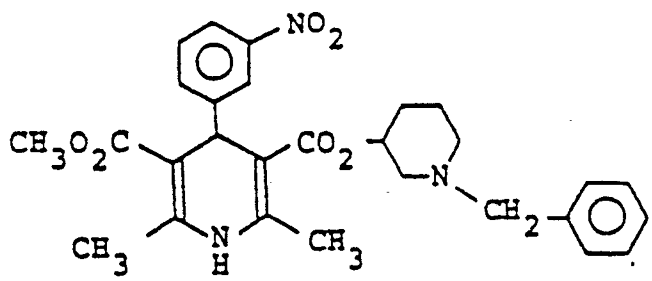

(I)

et de soumission dudit diastéréoisomère à une cristallisation fractionnée.

3. Procédé de préparation d'un composé tel que défini dans la revendication 1, qui comprend les étapes de réaction d'un composé représenté par la formule V :

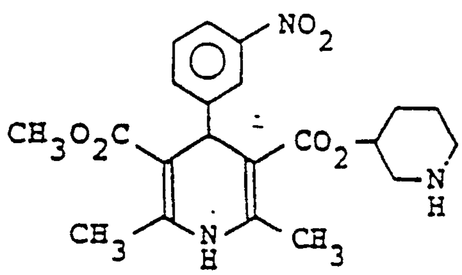

(V)

avec un composé représenté par la formule VI :

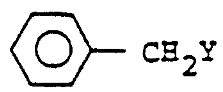

(VI)

dans laquelle Y représente un atome d'halogène, pour donner un diastéréoisomère représenté par la formule I et de soumission dudit diastéréoisomère à une cristallisation fractionnée.

4. Procédé de préparation d'un composé tel que défini dans la revendication 1, qui comprend les étapes de réaction d'un composé représenté par la formule VII :

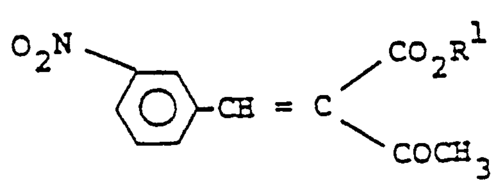

(VII)

avec un composé représenté par la formule VIII :

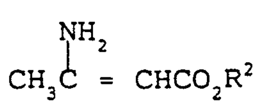

(VIII)

dans laquelle l'un de $R_1$ et $R_2$ représente un groupe méthyle et l'autre représente un groupe benzylpipéridyle, pour donner un diastéréoisomère représenté par la formule I et de soumission dudit diastéréoisomère à une cristallisation fractionnée.

5. Procédé de préparation d'un composé tel que défini dans la revendication 1, qui comprend les étapes

de réaction d'un composé représenté par la formule VII :

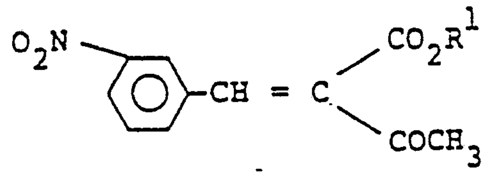

(VII)

avec un composé représenté par la formule IX :

$$CH_3COCH_2CO_2R^2 \quad (IX)$$

dans laquelle $R_1$ et $R_2$ ont les mêmes significations que celles définies ci-dessus, et avec $NH_3$ ou un sel d'ammonium, pour donner un diastéréoisomère représenté par la formule I et de soumission dudit diastéréoisomère à une cristallisation fractionnée.

6. Procédé de préparation d'un composé tel que défini dans la revendication 1, qui comprend les étapes de réaction d'un composé représenté par la formule X :

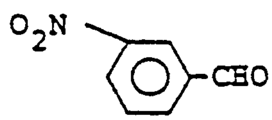

(X)

avec un composé représenté par la formule XI :

$$CH_3COCH_2CO_2R^1 \quad (XI)$$

et avec un composé représenté par la formule VIII

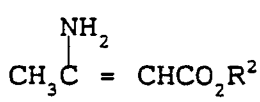

(VIII)

dans lesquelles $R_1$ et $R_2$ ont les mêmes significations que celles définies ci-dessus, pour donner un diastéréoisomère représenté par la formule I et de soumission dudit diastéréoisomère à une cristallisation fractionnée.

7. Isomère optique présentant un pouvoir rotatoire positif, de l'ester 5-méthylique et 3-(1-benzyl-3-pipéridylique) de l'acide (±)-2,6-diméthyl-4-(3-nitrophényl)-1,4-dihydropyridine-3,5-dicarboxylique, dont le chlorhydrate a un point de fusion de 196 à 202° C, ou de l'un de ses sels.

8. Procédé de préparation d'un composé tel que défini dans la revendication 7, qui comprend les étapes de réaction d'un composé représenté par la formule XIII:

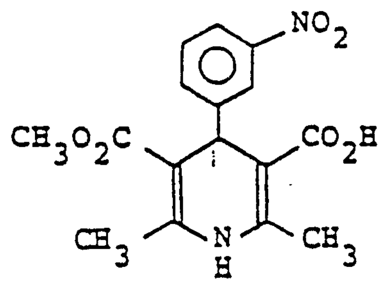

(XIII)

avec un composé représenté par la formule XIV :

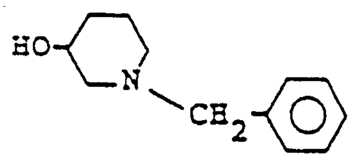

(XIV)

pour donner un composé représenté par la formule XII :

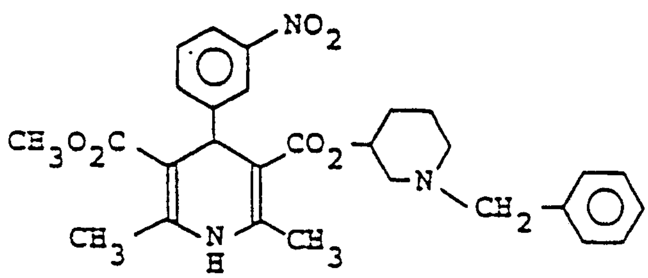

(XII)

et d'isolement d'un composé tel que défini dans la revendication 7 à partir du mélange réactionnel de façon classique, dans le cas où le Composé XIII et le Composé XIV sont tous deux des substances optiquement actives, ou par chromatographie sur colonne dans le cas où soit le Composé XIII, soit le Composé XIV, est un racémique.

**9.** Composition pharmaceutique comprenant un véhicule pharmaceutique et, en tant qu'ingrédient actif, une quantité efficace d'un composé tel que défini dans la revendication 1.

**10.** Composition pharmaceutique comprenant un véhicule pharmaceutique et, en tant qu'ingrédient actif, une quantité efficace d'un composé tel que défini dans la revendication 7.

Revendications Pour l'Etat Contractant AT

**1.** Procédé de préparation d'un ester 5-méthylique et 3-(1-benzyl-3-pipéridylique) de l'acide (±)-2,6-diméthyl-4-(3-nitrophényl)-1,4-dihydropyridine-3,5-dicarboxylique, dont le chlorhydrate a un point de fusion de 196 à 202° C, ou de l'un de ses sels, qui comprend les étapes de réaction d'un composé représenté par la formule II :

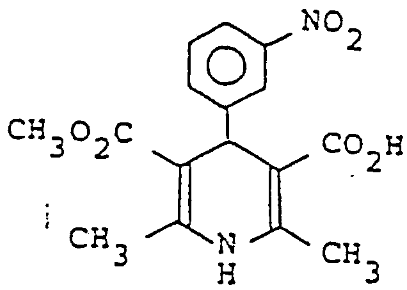

(II)

avec un composé représenté par la formule III :

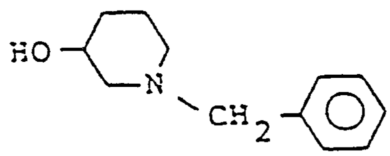

(III)

pour donner un diastéréoisomère représenté par la formule I :

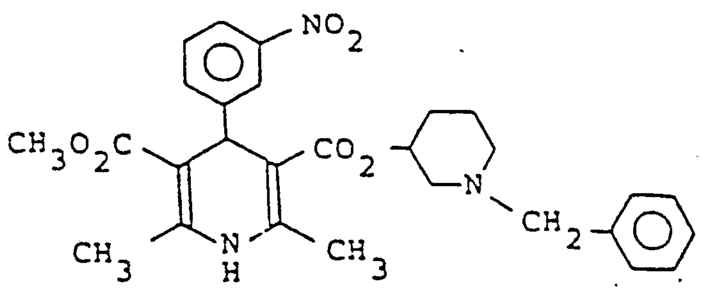

(I)

et de soumission dudit diastéréoisomère à une cristallisation fractionnée.

2. Procédé de préparation d'un ester 5-méthylique et 3-(1-benzyl-3-pipéridylique) de l'acide (±)-2,6-diméthyl-4-(3-nitrophényl)-1,4-dihydropyridine-3,5-dicarboxylique, dont le chlorhydrate a un point de fusion de 196 à 202° C, ou de l'un de ses sels, qui comprend les étapes de réaction d'un composé représenté par la formule V :

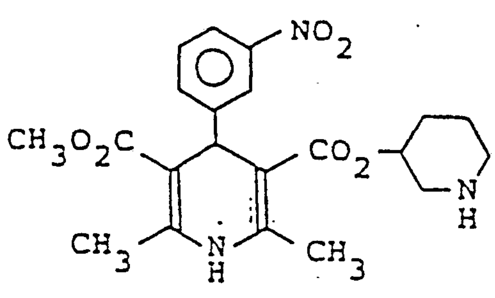

(V)

avec un composé représenté par la formule VI :

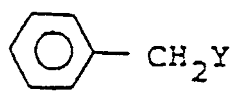

(VI)

dans laquelle Y représente un atome d'halogène, pour donner un diastéréoisomère représenté par la formule I et de soumission dudit diastéréoisomère à une cristallisation fractionnée.

3. Procédé de préparation d'un ester 5-méthylique et 3-(1-benzyl-3-pipéridylique) de l'acide (±)-2,6-diméthyl-4-(3-nitrophényl)-1,4-dihydropyridine-3,5-dicarboxylique, dont le chlorhydrate a un point de fusion de 196 à 202° C, ou de l'un de ses sels, qui comprend les étapes de réaction d'un composé représenté par la formule VII :

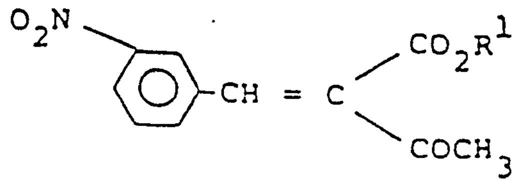

(VII)

avec un composé représenté par la formule VIII :

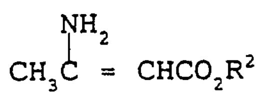

(VIII)

dans laquelle l'un de $R_1$ et $R_2$ représente un groupe méthyle et l'autre un groupe benzylpipéridyle, pour donner un diastéréoisomère représenté par la formule I

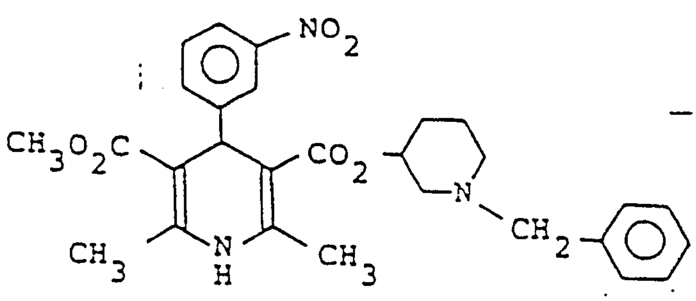

(I)

et de soumission dudit diastéréoisomère à une cristallisation fractionnée.

4. Procédé de préparation d'un ester 5-méthylique et 3-(1-benzyl-3-pipéridylique) de l'acide (±)-2,6-diméthyl-4-(3-nitrophényl)-1,4-dihydropyridine-3,5-dicarboxylique, dont le chlorhydrate a un point de fusion de 196 à 202° C, ou de l'un de ses sels, qui comprend les étapes de réaction d'un composé représenté par la formule VII :

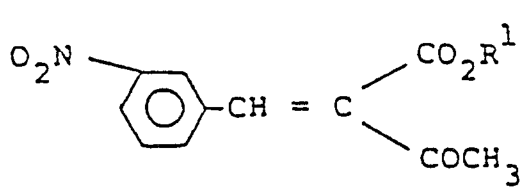

(VII)

avec un composé représenté par la formule IX :

$CH_3COCH_2CO_2R^2$ (IX)

dans laquelle $R_1$ et $R_2$ ont les mêmes significations que celles définies ci-dessus, et avec $NH_3$ ou un sel d'ammonium, pour donner un diastéréoisomère représenté par la formule I

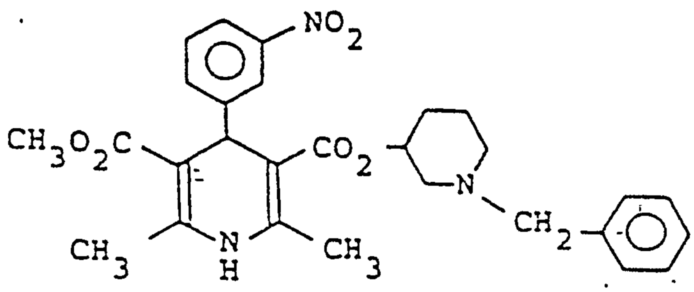

(I)

et de soumission dudit diastéréoisomère à une cristallisation fractionnée.

**5.** Procédé de préparation d'un ester 5-méthylique et 3-(1-benzyl-3-pipéridylique) de l'acide (±)-2,6-diméthyl-4-(3-nitrophényl)-1,4-dihydropyridine-3,5-dicarboxylique, dont le chlorhydrate a un point de fusion de 196 à 202° C, ou de l'un de ses sels, qui comprend les étapes de réaction d'un composé représenté par la formule X :

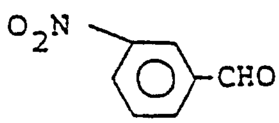

(X)

avec un composé représenté par la formule XI :

$CH_3COCH_2CO_2R^1$ (XI)

et avec un composé représenté par la formule VIII

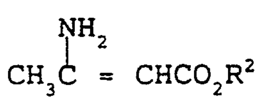

(VIII)

dans lesquelles $R_1$ et $R_2$ ont les mêmes significations que celles définies ci-dessus, pour donner un diastéréoisomère représenté par la formule I

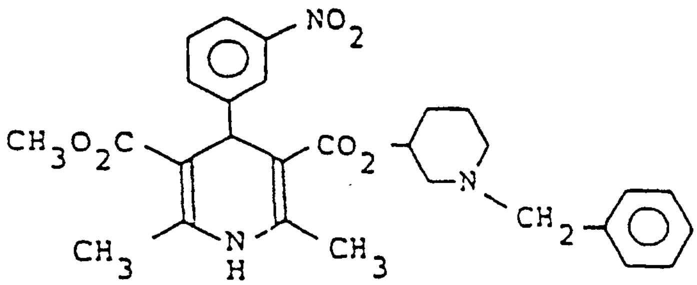

(I)

et de soumission dudit diastéréoisomère à une cristallisation fractionnée.

6. Procédé de préparation d'un isomère optique, présentant un pouvoir rotatoire positif, de l'ester 5-méthylique et 3-(1-benzyl-3-pipéridylique) de l'acide (±)-2,6-diméthyl-4-(3-nitrophényl)-1,4-dihydropyridine-3,5-dicarboxylique, dont le chlorhydrate a un point de fusion de 196 à 202° C, ou de l'un de ses sels, qui comprend les étapes de réaction d'un composé représenté par la formule XIII :

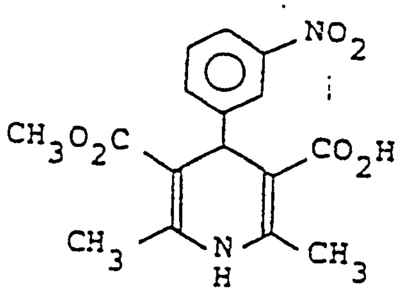

(XIII)

avec un composé représenté par la formule XIV :

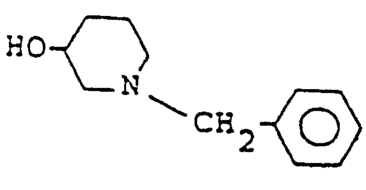

(XIV)

pour donner un composé représenté par la formule XII :

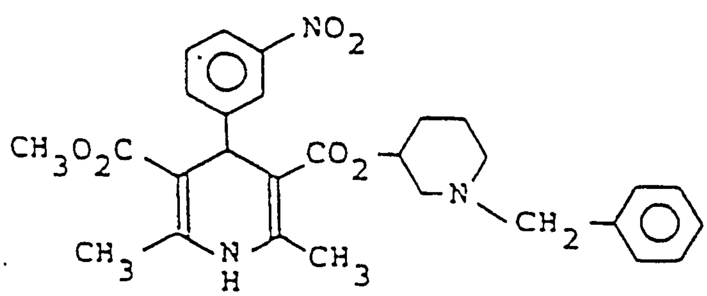

(XII)

et d'isolement dudit isomère optique présentant un pouvoir rotatoire positif à partir du mélange réactionnel de façon classique, dans le cas où le Composé XIII et le Composé XIV sont tous deux des substances optiquement actives, ou par chromatographie sur colonne dans le cas où soit le Composé XIII, soit le Composé XIV, est un racémique.

7. Procédé de préparation de la forme (±)-α souhaitée du chlorhydrate de l'ester 5-méthylique et 3-(1-benzyl-3-pipéridylique) de l'acide (±)-2,6-diméthyl-4-(3-nitrophényl)-1,4-dihydropyridine-3,5-dicarboxylique, présentant un point de fusion de 196 à 202° C, qui comprend la soumission du mélange réactionnel obtenu selon un procédé conforme à l'une quelconque des revendications 1 à 5 à une extraction, une concentration et/ou une chromatographie sur colonne, puis addition de chlorure d'hydrogène gazeux ou d'un chlorhydrate dans un solvant convenable pour former le chlorhydrate, et après l'élimination du solvant, si nécessaire, la réalisation d'une cristallisation fractionnée dans un solvant simple ou mixte convenable, au cours de laquelle la forme (±)-α se sépare par cristallisation et la forme (±)-β reste dans la solution.

8. Procédé conforme à la revendication 7, dans lequel on utilise, comme mélange solvant pour la cristallisation fractionnée, un mélange éthanol/acétone ou chloroforme/acétone.

9. Procédé de préparation d'une composition pharmaceutique, comprenant les étapes de préparation d'un ester 5-méthylique et 3-(1-benzyl-3-pipéridylique) de l'acide (±)-2,6-diméthyl-4-(3-nitrophényl)-1,4-dihydropyridine-3,5-dicarboxylique, dont le chlorhydrate a un point de fusion de 197 à 202° C, ou de l'un de ses sels, conformément à l'une quelconque des revendications 1 à 7, et le mélange d'une quantité efficace dudit composé avec un véhicule pharmaceutique.

## Ansprüche

1. (±)-2,6-Dimethyl-4-(3-nitrophenyl)-l,4-dihydropyridin-3,5-dicarbonsäure-3-(1-benzyl-3-piperidyl)-ester-5-methylester, wobei der Schmelzpunkt des Hydrochlorids Hievon 196° C bis 202° C beträgt, oder ein Salz hievon.

2. Verfahren zur Herstellung einer Verbindung, wie in Anspruch 1 definiert, welches die Stufen der et Umsetzung einer Verbindung, dargestellt durch die Formel II:

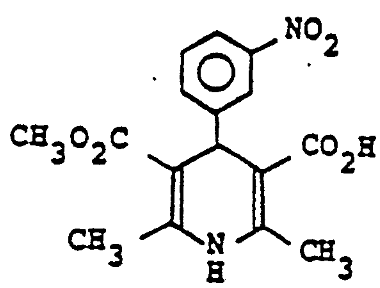

(II),

mit einer Verbindung, dargestellt durch die Formel III:

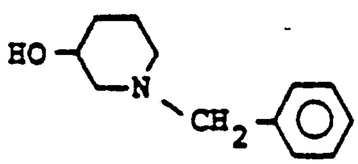

(III),

zu eine Diasteromer, dargestellt durch die Formel I:

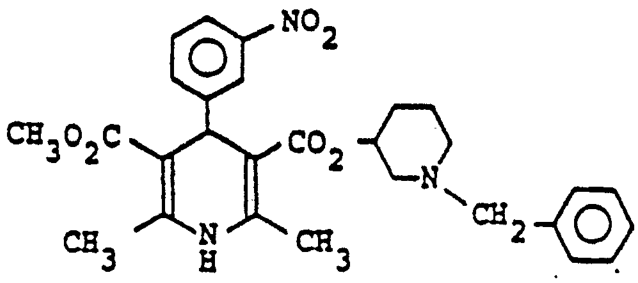

(I),

und Ausführung einer fraktionierten Kristallisation an diesem Diastereomer umfaßt.

3. Verfahren zur Herstellung einer Verbindung, wie in Anspruch 1 definiert, welches die Stufen einer Umsetzung einer Verbindung, dargestellt durch die Formel V:

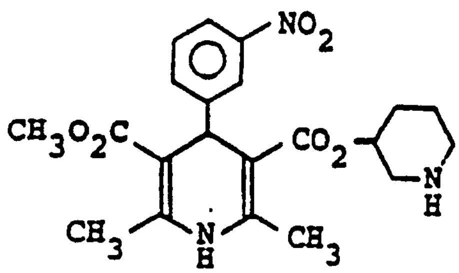

(V),

mit einer Verbindung, dargestellt durch die Formel VI:

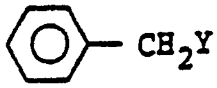

(VI),

worin Y ein Halogenatom bedeutet, zu einem durch die Formel I dargestellten Diastereomer und die Ausführung einer fraktionierten Kristallisation an diesem Diastereomer umfaßt.

4. Verfahren zur Herstellung einer Verbindung, wie in Anspruch 1 definiert, welches die Stufen des Umsetzens einer Verbindung, dargestellt durch die Formel VII:

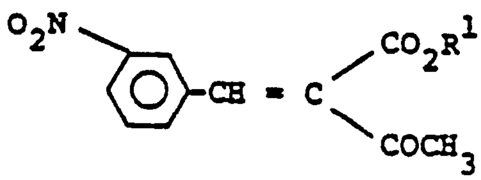

(VII),

mit einer Verbindung, dargestellt durch die Formel VIII:

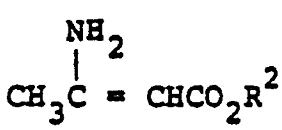

(VIII),

worin einer der Reste $R_1$ und $R_2$ eine Methylgruppe bedeutet und der andere Rest eine Benzylpiperidylgruppe darstellt, zu einem durch die Formel I dargestellten Diastereomer und das Ansführen einer fraktionierten Kristallisation an diesem Diastereomer umfaßt.

**5.** Verfahren zur Herstellung einer Verbindung, wie in Anspruch 1 definiert, welches die Stufen einer Umsetzung einer Verbindung, dargestellt durch die Formel VII:

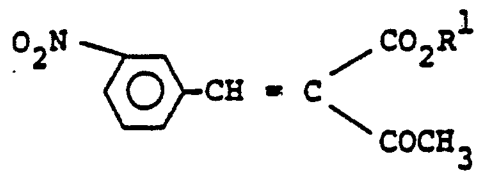

(VII),

mit einer Verbindung, dargestellt durch die Formel IX:

$$CH_3COCH_2CO_2R^2 \qquad (IX),$$

worin $R_1$ und $R_2$ die gleichen Bedeutungen wie vorstehend definiert aufweisen, und mit $NH_3$ (oder einem Ammoniunsalz) zu einem durch die Formel I dargestellten Diastereomer und das Ausführen einer fraktionierten Kristallisation an diesem Diastereomer umfaßt.

**6.** Verfahren zur Herstellung einer Verbindung, wie in Anspruch 1 definiert, welches die Stufen einer Umsetzung einer Verbindung, dargestellt durch die Formel X:

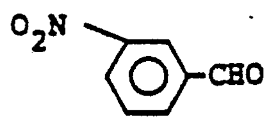

(X),

mit einer Verbindung, dargestellt durch die Formel XI:

$$CH_3COCH_2CO_2R^1 \qquad (XI),$$

und mit einer Verbindung, dargestellt durch die Formel VIII:

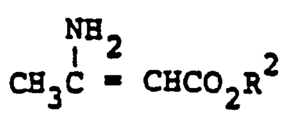

(VIII),

worin $R_1$ und $R_2$ die gleichen Bedeutungen wie vorstehend definiert aufweisen, zu einem durch die Formel I dargestellten Diastereomer und das Ausführen einer fraktionierten Kristallisation an diesem Diastereomer umfaßt.

**7.** Ein optisches Isomer mit einem (+)-optischen Rotationsgrad aus (±)-2,6-Dimethyl-4-(3-nitrophenyl)-1,4-dihydropyridin-3,5-dicarbonsäure-3-(1-benzyl-3-piperidyl)ester-5-methylester, dessen Hydrochlorid Schmelzpunkte von 196° C bis 202° C aufweist, oder ein Salz hievon.

**8.** Verfahren zur Herstellung einer Verbindung, wie in Anspruch 7 definiert, welches die Stufen des Umsetzens einer Verbindung, dargestellt durch die Formel XIII:

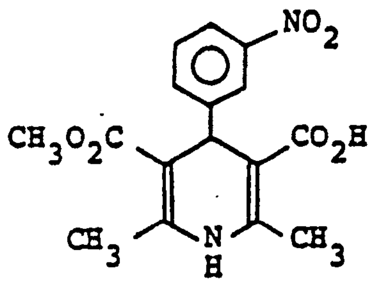

(XIII),

mit einer Verbindung, dargestellt durch die Formel XIV:

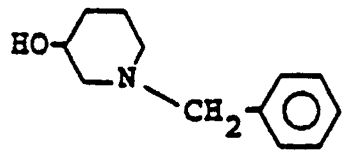

(XIV),

zu einer Verbindung, dargestellt durch die Formel XII:

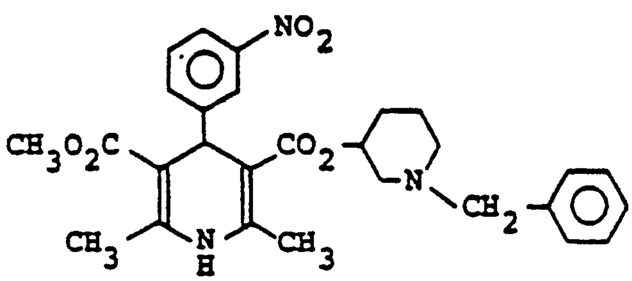

(XII),

und Isolieren einer wie in Anspruch 7 definierten Verbindung aus dem Reaktionsgemisch in üblicher Weise, falls die Verbindungen XIII und XIV beide optisch aktive Substanzen sind, oder durch Säulenchromatographie, falls entweder die Verbindung XIII oder die Verbindung XIV ein Racemat ist, umfaßt.

9. Pharmazeutische Zusammensetzung, umfassend einen pharmazeutischen Träger und als aktiven Bestandteil eine wirksame Menge einer Verbindung, wie in Anspruch 1 definiert.

10. Pharmazeutishe Zusammensetzung, umfassend einen pharmazeutischen Träger und als aktiven Bestandteil eine wirksame Menge einer Verbindung, wie in Anspruch 7 definiert.

Patentansprüche für den Vertragsstaat AT

1. Verfahren zur Herstellung eines (±)-2,6--Dimethyl-4-(3-nitrophenyl)-1,4-dihydropyridin-3,5-dicarbonsäure-3-(1-benzyl-3-piperidyl)ester-5-methylesters, dessen Hydrochlorid einen Schmelzpunkt von 196° C bis 202° C hat, oder eines Salzes hievon, welches Verfahren die Stufen der Umsetzung einer Verbindung, dargestellt durch die Formel II:

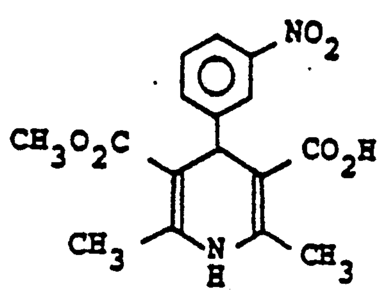

(II),

mit einer Verbindung, dargestellt durch die Formel III:

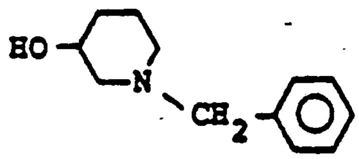

(III),

zu einem Diastereomer, dargestellt durch die Formel I:

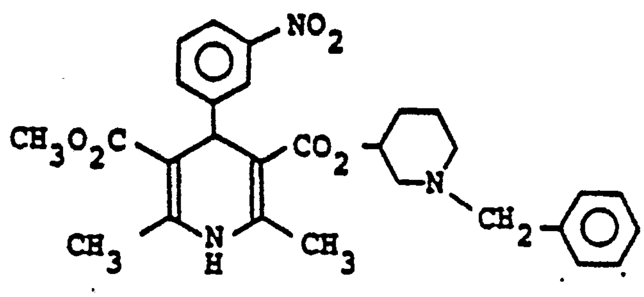

(I),

und Ausführung einer fraktionierten Kristallisation an diesem Diastereomer umfaßt.

2. Verfahren zur Herstellung eines (±)-2,6-Dimethyl-4-(3-nitrophenyl)-1,4-dihydropyridin-3,5-dicarbonsäure-3-(1-benzyl-3-piperidyl)ester-5-methylesters, dessen Hydrochlorid einen Schmelzpunkt von 196° C bis 202° C hat, oder eines Salzes hievon, welches Verfahren die Stufen einer Umsetzung einer Verbindung, dargestellt durch die Formel V:

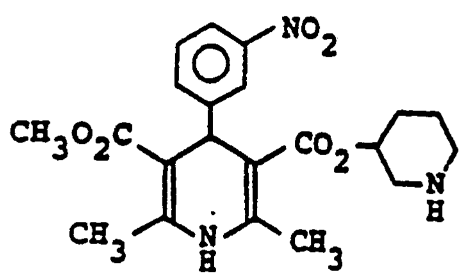

(V),

mit einer Verbindung, dargestellt durch die Formel VI:

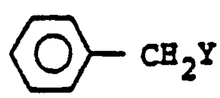

(VI),

worin Y ein Halogenatom bedeutet, zu einem durch die Formel I dargestellten Diastereomer und die Ausführung einer fraktionierten Kristallisation an diesem Diastereomer umfaßt.

3. Verfahren zur Herstellung eines (±)-2,6-Dimethyl-4-(3-nitrophenyl)-1,4-dihydropyridin-3,5-dicarbonsäure-3-(1-benzyl-3-piperidyl)ester-5-methylesters, dessen Hydrochlorid einen Schmelzpunkt von 196° C bis 202° C hat, oder eines Salzes hievon, welches Verfahren die Stufen des Umsetzens einer Verbindung, dargestellt durch die Formel VII:

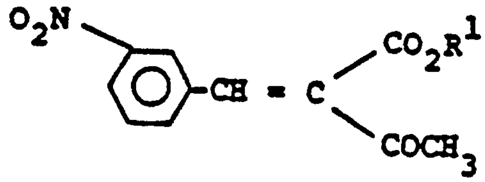

(VII),

mit einer Verbindung, dargestellt durch die Formel VIIII:

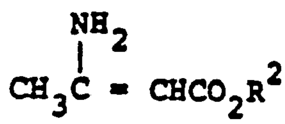

(VIII),

worin einer der Reste $R_1$ und $R_2$ eine Methylgruppe bedeutet und der andere Rest eine Benzylpiperidylgruppe darstellt, zu einem durch die Formel I:

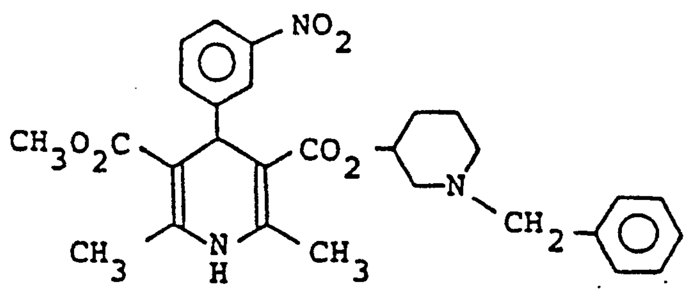

( I )

dargestellten Diastereomer und das Ausführen einer fraktionierten Kristallisation an diesem Diastereomer umfaßt.

4. Verfahren zur Herstellung eines (±)-2,6-Dimethyl-4-(3-nitrophenyl)-1,4-dihydropyridin-3,5-dicarbonsäure-3-(1-benzyl-3-piperidyl)ester-5-methylesters, dessen Hydrochlorid einen Schmelzpunkt von 196° C bis 202° C hat, oder eines Salzes hievon, welches Verfahren die Stufen einer Umsetzung einer Verbindung, dargestellt durch die Formel VII:

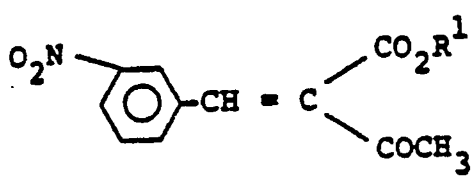

(VII),

mit einer Verbindung, dargestellt durch die Formel IX:

$$CH_3COCH_2CO_2R^2 \quad \text{(IX)},$$

worin $R_1$ und $R_2$ die gleichen Bedeutungen wie vorstehend definiert aufweisen, und mit $NH_3$ (oder einem Ammoniumsalz) zu einem durch die Formel I

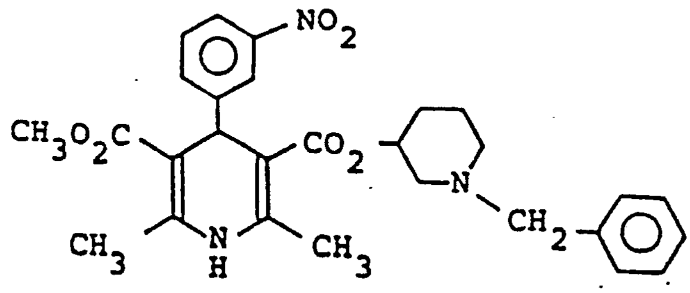

( I )

dargestellten Diastereomer und das Ausführen einer fraktionierten Kristallisation an diesem Diastereomer umfaßt.

**5.** Verfahren zur Herstellung eines (±)-2,6-Dimethyl-4-(3-nitrophenyl)-1,4-dihydropyridin-3,5-dicarbonsäure-3-(1-benzyl-3-piperidyl)ester-5-methylesters, dessen Hydrochlorid einen Schmelzpunkt von 196° C bis 202° C hat, oder eines Salzes hievon, welches Verfahren die Stufen einer Umsetzung einer Verbindung, dargestellt durch die Formel X:

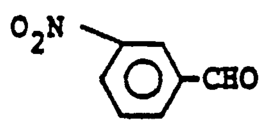

(X),

mit einer Verbindung, dargestellt durch die Formel XI:

$$CH_3COCH_2CO_2R^1 \qquad (XI),$$

und mit einer Verbindung, dargestellt durch die Formel VIII:

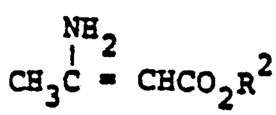

(VIII),

worin $R_1$ und $R_2$ die gleichen Bedeutungen wie vorstehend definiert aufweisen, zu einem durch die Formel I

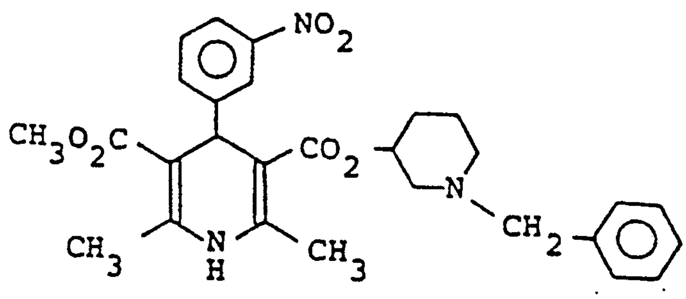

( I )

dargestellten Diastereomer und das Ausführen einer fraktionierten Kristallisation an diesem Diastereomer umfaßt.

6. Verfahren zur Herstellung eines optischen Isomers mit einem (+)-optischen Rotationsgrad aus (±)-2,6--Dimethyl-4-(3-nitrophenyl)-1,4-dihydropyridin-3,5-dicarbonsäure-3-(1-benzyl-3-piperidyl)ester-5-5-methylester, dessen Hydrochlorid Schmelzpunkte von 196° C bis 202° C aufweist, oder eines Salzes hievon, welches Verfahren die Stufen des Umsetzens einer Verbindung, dargestellt durch die Formel XIII:

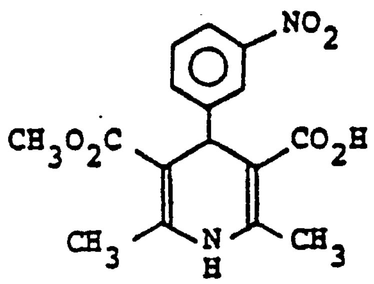

(XIII),

mit einer Verbindung, dargestellt durch die Formel XIV:

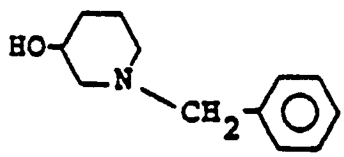

(XIV),

zu einer Verbindung, dargestellt durch die Formel XII:

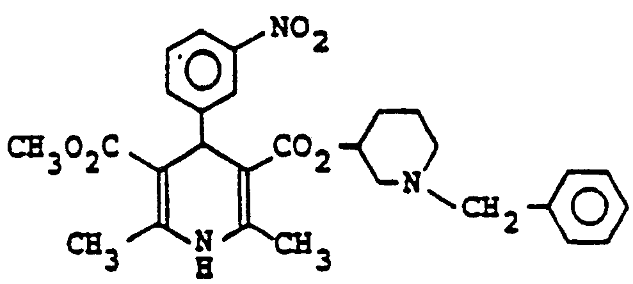

(XII),

und Isolieren dieses optischen Isomers mit einem (+)-optischen Rotationsgrad aus dem Reaktionsgemisch in üblicher Weise, falls die Verbindungen XIII und XIV beide optisch aktive substanzen sind, oder durch Säulenchromatographie, falls entweder die Verbindung XIII oder die Verbindung XIV ein Racemat ist, umfaßt.

7. Verfahren zur Herstellung der gewünschten (±)-α-Form des 2,6-Dimethyl-4-(3-nitrophenyl)-1,4-dihydropyridin-3,5-dicarbonsäure-3-(1-benzyl-3-piperidyl)-ester-5-methylester-Hydrochlorids mit einem Schmelzpunkt von 196° C bis 202° C, welches Verfahren die Ausführung einer Extraktion, Konzentration und/oder Säulenchromatographie an dem nach einem Verfahren gemäß einem der Ansprüche 1 bis 5 erhaltenen Reaktionsgemisch, die anschließende Zugabe von Chlorwasserstoffgas oder Chlorwasserstoffsalz in einem geeigneten Lösungsmittel zur Ausbildung des Hydrochlorids und nach Abtrennung des Lösungsmittels, soferne erforderlich, die Ausführung einer fraktionierten Kristallisation aus einem geeigneten einfachen oder gemischten Lösungsmittel umfaßt, wodurch die (±)-α-Form auskristallisiert wird und die (±)-β-Form in Lösung bleibt.

8. Verfahren nach Anspruch 7, worin Ethanol-Aceton oder Chloroform-Aceton als gemischte Lösungsmittel für die fraktionierte Kristallisation verwendet werden.

9. Verfahren zur Herstellung einer pharmazeutischen Zusammensetzung, umfassend die Stufen der Herstellung eines (±)-2,6-Dimethyl-4-(3-nitrophenyl)-1,4-dihydropyridin-3,5-dicarbonsäure-3-(1-benzyl-3-piperidyl)-ester-5-methylesters, dessen Hydrochlorid einen Schmelzpunkt von 196° C bis 202° C aufweist, oder eines Salzes hievon nach einem der Ansprüche 1 bis 7 und das Vermischen einer wirksamen Menge dieser Verbindung mit einem pharmazeutischen Träger.